# EUROPEAN PATENT SPECIFICATION

(11) **EP 1 605 974 B1**
(45) Date of publication and mention of the grant of the patent: **12.11.2014**
(21) Application number: 04717386.9
(22) Date of filing: 04.03.2004
(51) Int. Cl.: A61K 39/395, A61K 39/40, A61K 39/42, C07K 16/00

(54) **METHOD OF TREATING AUTOIMMUNE DISEASE BY INDUCING ANTIGEN PRESENTATION BY TOLERANCE INDUCING ANTIGEN PRESENTING CELLS**
VERFAHREN ZUR BEHANDLUNG VON AUTOIMMUNKRANKHEITEN DURCH INDUKTION DER ANTIGENPRÄSENTATION DURCH TOLERANZ-INDUZIERENDE ANTIGEN-PRÄSENTIERENDE ZELLEN
PROCEDE DE TRAITEMENT DE MALADIES AUTO-IMMUNES EN SUSCITANT UNE PRESENTATION D'ANTIGENES PAR DES CELLULES PRESENTANT DES ANTIGENES INDUCTRICES DE TOLERANCE

(30) Priority: 04.03.2003 US 451816 P; 15.12.2003 US 529500 P; 28.02.2004 US 548385 P
(43) Date of publication of application: 21.12.2005
(73) Proprietor: ALEXION PHARMACEUTICALS, INC., Cheshire, CT 06410 (US)
(72) Inventor: BOWDISH, Katherine, S., Del Mar, CA 92014 (US); KRETZ-ROMMEL, Anke, San Diego, CA 92116 (US); DAKAPPAGARI, Naveen, San Diego, CA 92130 (US)
(74) Representative: Vossius & Partner
(86) International application number: PCT/US2004/006570
(87) International publication number: WO 2004/091543

(56) References cited:
- LUNDE E ET AL: "Troybodies and pepbodies" BIOCHEMICAL SOCIETY TRANSACTIONS, COLCHESTER, ESSEX, GB, vol. 30, no. PART 4, 2002, pages 500-506, XP002227237 ISSN: 0300-5127
- HAWIGER D ET AL: "Dendritic cells induce peripheral T cell unresponsiveness under steady state conditions in vivo" JOURNAL OF EXPERIMENTAL MEDICINE, TOKYO, JP, vol. 194, no. 6, 17 September 2001 (2001-09-17), pages 769-779, XP002376445 ISSN: 0022-1007
- SCHJETNE K W ET AL: "A MOUSE CK-SPECIFIC T CELL CLONE INDICATES THAT DC-SIGN IS AN EFFICIENT TARGET FOR ANTIBODY MEDIATED DELIVERY OF T CELL EPITOPES FOR MHC CLASS II PRESENTATION" INTERNATIONAL IMMUNOLOGY, OXFORD UNIVERSITY PRESS, GB, vol. 14, 2002, pages 1423-1430, XP008043258 ISSN: 0953-8178
- LUNDE E. ET AL.: 'Efficient delivery of T cell epitopes to APC by use of MHC class II-specific troybodies' J. IMMUNOL. vol. 168, 2002, pages 2154 - 2162, XP002983188
- BONIFAZ L. ET AL.: 'Efficient targeting of protein antigen to the dendritic cell receptor DEC-205 in the steady state leads to antigen presentation on major histocompatibility complex class I products and peripheral CD8+ T cell tolerance' J. EXP. MED. vol. 196, no. 12, 16 December 2002, pages 1627 - 1638, XP002983189
- KUWANA M.: 'Induction of anergic and regulatory T cell by plasmacytoid dendritic cells and other dendritic cell subsets' HUM. IMMUNOL. vol. 63, 2002, pages 1156 - 1163, XP002978586
- GILLIET M. ET AL.: 'HUamn plasmacytoid-derived dendritic cells and the induction of T-regulatory cells' HUM. IMMUNOL. vol. 63, 2002, pages 1149 - 1155, XP002983190
- LIMMER ANDREAS ET AL: "Efficient presentation of exogenous antigen by liver endothelial cells to CD8+ T cells results in antigen-specific T-cell tolerance", NATURE MEDICINE, vol. 6, no. 12, December 2000 (2000-12), pages 1348-1354, ISSN: 1078-8956
- DAKAPPAGARI N ET AL: "Internalizing antibodies to the C-type lectins, L-SIGN and DC-SIGN, inhibit viral glycoprotein binding and deliver antigen to human dendritic cells for the induction of T cell responses", THE JOURNAL OF IMMUNOLOGY, THE AMERICAN ASSOCIATION OF IMMUNOLOGISTS, US, vol. 176, no. 1, 1 January 2006 (2006-01-01), pages 426-440, XP002385270, ISSN: 0022-1767
- KNOLLE P A ET AL: "Induction of cytokine production in naive CD4<+> T cells by antigen-presenting murine liver sinusoidal endothelial cells but failure to induce differentiation toward Th1 cells", GASTROENTEROLOGY, ELSEVIER, PHILADELPHIA, PA, vol. 116, no. 6, 1 June 1999 (1999-06-01), pages 1428-1440, XP027522267, ISSN: 0016-5085 [retrieved on 1999-06-01]

## Description

### Technical Field

Developing and restoring natural immune tolerance to autoantigens to treat or prevent autoimmune diseases.

### Background of Related Art

T cell-mediated disease insulin-dependent diabetes mellitus ("T1DM") is a major health problem, affecting more than 1.5 million Americans. This autoimmune disease results from the T cell-mediated destruction of insulin-producing β-cells of the islets of Langerhans within the pancreas. Despite treatment with insulin, deaths resulting from T1 DM have increased in the past 20 years, whereas mortality from cancer, cardiovascular disease and stroke have decreased (Hurlbert et al, 2001). In addition, complications of treatment with exogenous insulin including nephropathy, neuropathy and retinopathy are very debilitating.

T1DM is considered a Th1-mediated disease and early intervention which shifts the immune response towards a Th2 type, for example by systemic administration of IL-4, can prevent onset of disease (Cameron et al, 1997). The balance of the effector T cells, Th1 and Th2, may be important in maintaining immune tolerance, and shift in balance can result in autoimmunity. However, protection from autoimmune disease is not an intrinsic property of Th2 cells since Th2 cell lines from NOD mice have also been shown to transfer disease (Pakkala et al, 1997).

The immune system has evolved in complex ways to maintain self-tolerance. The thymus provides an important initial selection of T cells. This selection results in the export, to the periphery, of T-cells which are tolerant to self-antigens present in the thymus. However, many tissue-specific proteins are not expressed at sufficient levels to induce tolerance. For example, islet of Langerhans-reactive T cells have been found in healthy subjects, though presumably of low affinity (Lohman et al. 1996). Several mechanisms of peripheral tolerance complement central tolerance mechanisms in the thymus to keep autoreactive T cells under control. One of the key mediators of peripheral tolerance is the antigen presenting cell ("APC"). APCs such as dendritic cells ("DCs") and macrophages capture self antigens from other cells and present them to autoreactive T cells to induce T cell tolerance by deletion, anergy and/or generation of regulatory T cells (Heath & Carbone, 2001). The current hypothesis is that immature APCs, such as APCs in the steady-state immune system, tolerize rather that activate T cells presumably due to a lack of co-stimulatory molecules. Hawiger et al. have targeted antigen to the major histocompatibility class II ("MHC, II") pathway of DCs using antibodies to DEC-205, a DC-restricted endocyte receptor (Hawiger et al., 2001). The antigen presentation by these DCs prompted a short burst of CD4+ T cell proliferation, followed by deletion and recipients were rendered tolerant to the antigen, as shown by lack of response to subsequent peptide immunization. In contrast, when antigen targeting was accompanied by a strong DC maturation stimulus such as anti- CD40, immunity was induced.

Dendritic cells can also induce peripheral tolerance by generating regulatory T cells that influence the functions of effector T cells through suppressive cytokines or a contact-dependent mechanism (Roncarolo et al, 2001; Jonuleit et al, 2000; Dhodapkar & Steinman, 2001). A number of different protocols for the induction of regulatory T cells have been developed, generally by means of "suboptimal" T cell stimulation. Suboptimal stimulation of T cells can be accomplished by antigen presentation in the absence of co-stimulation, or inflammation, or by partial blocking of the T cell receptor or its co-receptors CD4 and CD8. The phenotype and mechanism of action of the regulatory T cells is heterogeneous. Many suppressor cells are CD4+CD25+, however it is becoming increasingly clear that in many situations CD4+CD25- cells are equally effective. Other markers identified in the regulatory T cell population include CD62L, GITR and CD103 (Lafaille & Lafaille, 2002), and CD8+ regulatory T cells have also been reported (Dhodapkar & Steinman, 2002). Some regulatory T cells have been shown to produce the immunosuppressive cytokine interleukin ("IL")-I0 (Wakkach et al, 2001; Barrat et all 2002), while regulatory T cells induced by oral tolerance have been characterized by the production of Transforming Growth Factor-β ("TGF-β"), in addition to the Th2 type cytokines IL-4 and IL-I0 (Weiner, 2001). Contact-dependent suppressor cells have been generated by activating CD4+CD45RA+ human peripheral T cells in the presence of TGF-β (Yamigawa et al, 2001). While induction of regulatory T cells requires stimulation through the T cell receptor, their suppressive effect appears to be non-antigen specific (Thorton & Shevach, 2000).

Immunoregulatory T cells have been shown to play a role in down modulating the pathogenic autoreactive T cells in NOD mice. There is evidence that prediabetic mice harbor immunoregulatory T cells and that a decrease in their numbers, or their functional capacity, is a major contributing event in the disease progression (Sempe et al, 1994). Co-transfer experiments have shown that CD4+ T splenocytes from prediabetic mice fully prevent disease transfer by diabetogenic cells into immuno-incompetent recipients (Boitard et al, 1989; Hutchings & Cooke, 1990). Also, induction of regulatory T cells by immature DCs correlated with disease prevention in the NOD mouse model (Huges et al, 2002).

In humans, autoreactive T cells responding to insulin, glutamic acid decarboxylase ("GAD"), heat shock protein ("HSP") 60, or protein tyrosine phosphatase-like molecule ("IA-2"), and other undefined β-cell antigens have been described (Roep et al, 1990; Atkinson et al, 1992; Honeyman et al, 1993; Reijonen et al, 2002).

GAD is a biosynthetic enzyme of the inhibitory neurotransmitter gamma animobutyric acid (Baekkeskov et al, 1990). Two distinct isoforms with 65% homology, GAD65 and GAD67, have been cloned. Although GAD65 is the predominant isoform in humans, whereas GAD67 is the major form in NOD mice, antibodies against both isoforms are detected in humans (Kaufman et al, 1992). In NOD mice, anti-GAD antibodies were detected before, or at the time of, insulitis, and before antibodies to other β-cell antigens developed. This timing implies that GAD is the primary antigen that initiates β-cell autoimmunity in this model (Tisch et al, 1993). Further evidence for an important role of GAD in diabetes comes from the observations by many laboratories that GAD-specific T cells isolated from spleen or pancreas of diabetic mice can transfer disease to naive animals (Rohane et al, 1995; Wen et al, 1998; Zekzer et al, 1998). Although there remains controversy with regard to the central role of GAD in the pathogenesis of T1DM, evidence from animal experiments suggests at least an important role of this protein.

Immunization with purified GAD65 at an early age either intrathymically or intravenously can tolerize T cells against pancreatic β-cells in NOD mice, thereby preventing insulitis and diabetes (Tian et al, 1996; Ma et al, 1997). Tolerization against GAD could also prevent the development of immune reactions against other antigens such as HSP65. Further studies addressed which GAD peptides were capable of inducing tolerance (Tisch et al, 2001; Tisch et al, 1999; Zechel et al, 1998). Protection from diabetes onset can also be achieved by either insulin or HSP65 treatment via the intravenous, subcutaneous, oral or nasal route (Elias et al, 1991; Elias & Cohen, 1994; Elias et all 1997; Atkinson et all 1990). While antigen-specific therapies are highly effective in preventing disease onset when administered early, only few attempts were successful at controlling ongoing disease (Elias & Cohen, 1994; Tian et al, 1996).

General peptide immunizations cannot control whether antigen presenting cells present the peptides at a stage that induces immunity or by antigen presenting cells that can shift the immune response towards tolerance, and therefore can result in either immune stimulation or immune suppression.

Compromising the immune system can prevent the development of diabetes. A vast array of general agents suppressing T cell function such as FK506, anti- CD4, anti-CD8, anti-CTLA-4 and others have been shown to prevent or delay diabetes onset in NOD mice (reviewed in: Atkinson & Leiter, 1999). However, none of these reagents is specific for diabetogenic T cells, and the majority of these can prevent onset of disease, but is ineffective once disease is established. General immunosuppressive agents such as cyclosporine tested in clinical trials have been effective short-term (Feutren et al, 1988; Skyler & Rabinovitch, 1992). However, discontinuation of immunosuppression led to prompt relapses, and side effects such as kidney toxicity preclude long-term treatment (Parving et al, 1999).

Clinical trials have been initiated to assess the efficacy of antigen-specific therapy in diabetes. The HSP6O p277 peptide (DiaPep277) was tested in early onset diabetics (Raz et al, 2001). Multiple immunizations with the peptide slowed the disease progression and large-scale studies have been initiated to validate and extend the results. Clinical trials using the beta-chain of human insulin in combination with incomplete Freund's adjuvant, an altered peptide ligand of insulin B9-23 and GAD, are underway. However, trials treating recently diagnosed diabetics with oral insulin failed (Pozzili et al. 2000; Chaillous et al. 2000) and parenteral insulin administration was unsuccessful in preventing disease in high risk prediabetics (Diabetes Prevention Trial-Type 1 (DPT) Study Group, 2002). Failure could be due to several factors including choice of antigen, antigen dose (Kurts et al., 1999), timing and route of administration. Also, antigen therapy can not control what type of immune cell takes up the antigen. While mice are under controlled pathogen-free conditions, this is not the case in human trials. Priming, rather than tolerance can take place when there are concurrent bacterial or viral infections. In animals, diabetes could be induced by antigen immunization under certain conditions (Blana et al. 1996; Bellmann et al. 1998).

Bonifaz et al., (J. Exp. Med. 196(12), 1627, 2002) discloses that construct comprising a DEC-205 antibody and ovalbumin can induce tolerance to ovalbumin. Since the understanding of how the immune system maintains tolerance to self-antigens has grown substantially in the past decade, current therapeutic strategies to prevent or cure T1 DM aim at restoring immune tolerance to β-cell antigens. Current immunotherapy strategies are aimed at inducing tolerance to β-cell antigens either by directly inactivating the autoreactive T cells and/or inducing T cells with regulatory capabilities. Induction of regulatory T cells appears to be a promising approach for treatment of a number of autoimmune diseases.

### Summary

The present invention is defined by the claims. In more detail, the present invention relates to an antibody/peptide construct comprising: (i) an antibody that recognizes L-SIGN and (ii) a peptide linked to the antibody, wherein the peptide is an autoantigen, wherein the autoantigen is selected from the group consisting of glutamic acid decarboxylase (GAD), an epitope of GAD, insulin, an epitope of insulin, heat shock protein (HSP), an epitope of HSP and β cell antigens. The present invention furthermore pertains to a method for recombinantly producing engineered antibodies, the method comprising linking an antibody that recognizes L-SIGN to an autoantigen, wherein the autoantigen is selected from the group consisting of glutamic acid decarboxylase (GAD), an epitope of GAD, insulin, an epitope of insulin, heat shock protein (HSP), an epitope of HSP and β cell antigens. Also described herein is a method of treating autoimmune disease by inducing immune tolerance. The immune tolerance is induced by presenting autoantigens onto antigen-presenting cells. The autoantigens are linked to antibodies which recognize antigen- internalizing receptors. The autoantigens are internalized by and presented on the antigen-presenting cells, causing an inhibition of autoreactive T cells.

The methods and compounds described herein are used to treat diabetes mellitus by inducing an immune tolerance to an autoantigen, which can be, inter alia, β cell antigens, GAD or an epitope thereof, insulin or an epitope thereof, HSP or an epitope thereof. The autoantigen is linked to an antibody to that recognizes DC-SIGNR, or a variation of DC-SIGNR, which is an antigen-internalizing receptor. The autoantigen is internalized into the target liver sinusoidal endothelial cells or other tolerizing APC's expressing DC-SIGNR on the surface. The autoantigen is presented on the target liver sinusoidal endothelial cells and inhibits the proliferation of autoreactive T cells.

Antibody/peptide constructs are described which contain an antibody to a receptor on an antigen presenting cell linked to a peptide. The peptide may be an antigen or may be an autoantigen. The antibody/autoantigen construct or portion thereof may be internalized by the antigen presenting cell and immune tolerance to the autoantigen is achieved. In some cases a toxin can be combined with the antibodies of the present disclosure and administered to a patient. Where the toxin is to, e.g., a tumor cell, the antibody of the present disclosure can be utilized to direct the toxin to the tumor cell and thereby focus administration of the toxin to the tumor cell.

Also described herein are methods for recombinantly producing engineered antibodies that contain an antibody to a receptor or an antigen presenting cell linked to an autoantigen are described.

The present disclosure also relates to antibodies to DC-SIGNR which interfere with the interaction of DC-SIGNR expressing cells and ICAM-expressing cells such as T cells.

The antibodies to DC-SIGNR may prevent entry of viruses into liver cells such as liver sinusoidal cells and their infection into other cells. The present disclosure may include the use of antibodies to DC-SIGNR in vaccines.

The antibodies to DC-SIGNR of the present disclosure can be a humanized antibody. The antibodies to DC-SIGNR of the present disclosure can also be an scFv.

Also described herein are prophylactic techniques as well as diagnostic techniques using the compositions and/or embodying the methods as described above. Compositions comprising the antibodies to DC-SIGNR of the present disclosure in a pharmaceutically acceptable carrier are also provided.

### Brief Description of the Drawings

Figure 1 schematically shows the interaction an antibody/autoantigen construct in accordance with the present disclosure with an antigen presenting cell (APC), and a T cell.
Figure 2A shows the light chain amino acid sequences (SEQ. ID NOS: 1-6) and heavy chain amino acid sequences of rabbit anti-mSIGNR1 scFV antibodies.
Figure 2B shows the heavy chain amino acid sequences (SEQ. ID NOS: 7-12) of rabbit anti-mSIGNR1 scFV antibodies.
Figure 3 is a schematic diagram of a portion of a vector for antibody peptide construct production.
Figure 4 is a graphical depiction of the results of in vitro experiments in accordance with the present disclosure showing the reactivity of IgG1 clones with human DC-SIGNR.
Figure 5 is a graphical depiction of the results of in vitro experiments in accordance with the present disclosure showing the reactivity of IgG2a clones with human DC-SIGNR.
Figure 6 is a graphical depiction of the results of in vitro experiments in accordance with the present disclosure showing the reactivity of IgG1 clones with human DC-SIGNR and DC-SIGN.
Figure 7 is a graphical depiction of the results of in vitro experiments in accordance with the present disclosure showing the reactivity of IgG2a clones with human DC-SIGNR and DC-SIGN.
Figures 8A-8C shows the amino acid sequences of heavy chain clones reactive with human DC-SIGNR (SEQ. ID NOS. 17-36).
Figures 9A-9B shows the amino acid sequences of light chain clones reactive with human DC-SIGNR (SEQ. ID NOS. 37-55).
Figure 10 shows additional amino acid sequences of heavy chain clones reactive with human DC-SIGNR (SEQ. ID NOS. 63-82).
Figure 11 shows additional amino acid sequences of light chain clones reactive with DC-SIGNR (SEQ. ID NOS. 46-226).
Figure 12 shows additional amino acid sequences of heavy chain clones reactive with DC-SIGNR (SEQ. ID NOS. 133-154).
Figure 13 shows additional amino acid sequences of light chain clones reactive with DC-SIGNR (SEQ. ID NOS. 169-189).

### Detailed Description

The present methods induce immune tolerance to autoantigens, or self-peptides, implicated in autoimmune disease.

Immunotolerance is induced in accordance with the present disclosure by administering an antibody/autoantigen construct (sometimes referred to herein as an "engineered antibody") to a subject. The antibody/autoantigen construct includes an autoantigen linked to an antibody.

The antibody component can be an antibody that binds to any receptor on any antigen presenting cell. As those skilled in the art will appreciate, types of antigen presenting cells include dendritic cells, macrophages, endothelial cells Kupffer cells and B cells. Among the presently known receptors or antigen presenting cells are DEC-205, mannose receptor, DC-SIGN, DC-SIGNR, MHC, toll receptor, langerin, asialoglycoprotien receptor, beta-glucan receptor, C-type lectin receptor and dendritic cell immunoreceptor. The receptor may be one that will internalize the STT antibody. Whether internalization occurs at a particular receptor can be determined experimentally using techniques known to those skilled in the art. Receptors or antigen presenting cells that are presently known to provide internalization of antibodies include DEC-205, mannose receptor, DC-SIGN and DC-SIGNR.

The antibody component can be a natural antibody (isolated using conventional techniques) or an antibody that is synthetically prepared by recombinant methods within the purview of those skilled in the art. The antibody can be, for example, a fully human antibody, a non-human antibody, a humanized antibody, a chimeric antibody or any of the foregoing types of antibodies that have been manipulated in any way (e.g., site-specific modifications or de-immunization). The antibody can be advantageously selected from a library of antibodies using techniques known to those skilled in the art, such as, for example phage display and panning.

Once selected, nucleic acid encoding the antibody can be amplified using techniques known to those skilled in the art such as, for example, conventional PCR or the amplification technique described in U.S. Patent Application Nos. 10/251,085 filed September 19, 2002 and 10/014,012 filed December 10, 2001, respectively.

An autoantigen is linked to the antibody to prepare an antibody/autoantigen construct in accordance with this disclosure. Any autoantigen can be employed. The autoantigen can be naturally occurring and isolated using techniques known to those skilled in the art. Alternatively, if the amino acid sequence of the autoantigen is known, it can be synthetically prepared using known techniques. Suitable autoantigens include insulin, GAD, Hsp, nuclear antigens, acetylcholine receptor, myelin basic protein, myelin oligodendrocyte glycoprotein, proteolipid protein, myelin associated glycoprotein, glomular basement membrane protein and thyrotropin receptor. The autoantigen may be one that induces immune tolerance upon presentation by an antigen presenting cell.

The autoantigen can be linked to the antibody by any suitable method. One particular method is set forth in the Examples, infra, however this disclosure is not limited to any particular method of making the antibody/autoantigen construct.

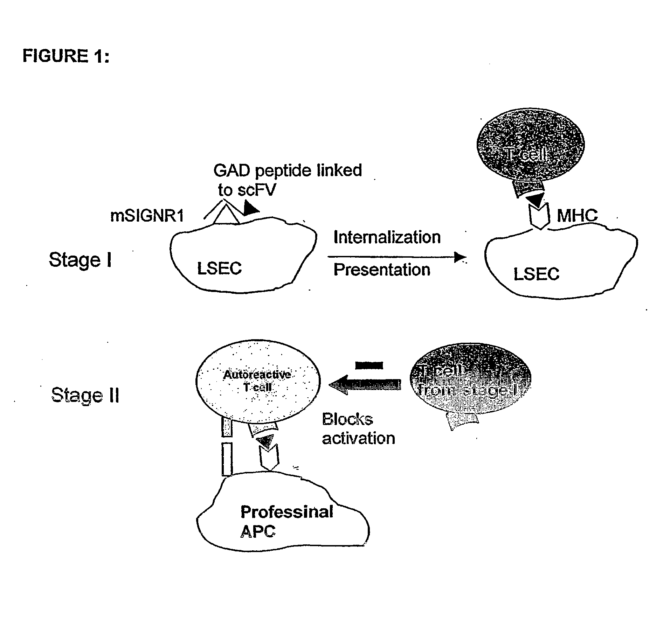

The present methods of inducing immune tolerance to autoantigens target antigen-presenting cells ("APCs") and direct an autoantigen to those cells by way of an antibody. Figure 1 schematically shows the interaction an antibody/autoantigen construct in accordance with the present disclosure with an antigen presenting cell (APC), and a T cell. The antibody recognizes a receptor on the targeted cells. To direct delivery of the autoantigen via the antibody, the two are linked. This linking may be accomplished by any method, although this disclosure delineates the use of vector cloning. The antibody targets and binds to the unique antigen-internalizing receptor only, thereby assuring delivery of the autoantigen to the desired cell type.

After the antibody is bound to the targeted antigen-internalizing receptor, the linked autoantigen and the antibody are internalized in the antigen presenting cell. The autoantigen is presented on the surface of the APCs, presumably through the autoantigen's interaction with major histocompatibility complex ("MHC") within the cell. Once an autoantigen is expressed on the surface of the APCs with co-stimulatory potential, naïve autoreactive T cells can become activated and target and react with their specific autoantigen. The absence of a co-stimulatory molecule in the surface of the APCs is most likely involved in limiting the T cell response. Autoreactive effector T cells can kill only a limited number of antigen expressing tissue cells. After killing a few target cells, the effector cell dies. The autoantigen presenting cells are then tolerated.

The described antibody/autoantigen construct can be administered in accordance with known methods, e.g., injection or infusion by intravenous, intraperitoneal, intracerebral, intramuscular, subcutaneous, intraocular, intraarterial, intrathecal, inhalation or intralesional routes, topical or by sustained release systems as noted below. The antibody/autoantigen construct may be administered continuously by infusion or by bolus injection. One may administer the antibody/autoantigen construct in a local or systemic manner.

The antibody/autoantigen constructs may be prepared in a mixture with a pharmaceutically acceptable carrier. Techniques for formulation and administration of the compounds of the instant application may be found in "Remington's Pharmaceutical Sciences," Mack Publishing Co., Easton, PA, latest edition. This therapeutic composition can be administered intravenously or through the nose or lung, preferably as a liquid or powder aerosol (lyophilized). The composition may also be administered parenterally or subcutaneously as desired. When administered systemically, the therapeutic composition should be sterile, pyrogen-free and in a parenterally acceptable solution having due regard for pH, isotonicity, and stability. These conditions are known to those skilled in the art.

Briefly, dosage formulations of the described antibody/autoantigen construct are prepared for storage or administration by mixing the compound having the desired degree of purity with physiologically acceptable carriers, excipients, or stabilizers. Such materials are non-toxic to the recipients at the dosages and concentrations employed, and may include buffers such as TRIS HCl, phosphate, citrate, acetate and other organic acid salts; antioxidants such as ascorbic acid; low molecular weight (less than about ten residues) peptides such as polyarginine, proteins such as serum albumin, gelatin, or immunoglobulins; hydrophilic polymers such as polyvinylpyrrolidinone; amino acids such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium and/or nonionic surfactants such as TWEEN, PLURONICS or polyethylene glycol.

When used for *in vivo* administration, the antibody/autoantigen construct formulation must be sterile and can be formulated according to conventional pharmaceutical practice. This is readily accomplished by filtration through sterile filtration membranes, prior to or following lyophilization and reconstitution. The antibody ordinarily will be stored in lyophilized form or in solution. Other vehicles such as naturally occurring vegetable oil like sesame, peanut, or cottonseed oil or a synthetic fatty vehicle like ethyl oleate or the like may be desired. Buffers, preservatives, antioxidants and the like can be incorporated according to accepted pharmaceutical practice.

Pharmaceutical compositions suitable for use include compositions wherein one or more antibody/autoantigen constructs are contained in an amount effective to achieve their intended purpose. More specifically, a therapeutically effective amount means an amount of antibody effective to prevent, alleviate or ameliorate symptoms of disease or prolong the survival of the subject being treated. Determination of a therapeutically effective amount is well within the capability of those skilled in the art, especially in light of the detailed disclosure provided herein. Therapeutically effective dosages may be determined by using *in vitro* and *in vivo* methods.

An effective amount of antibody/autoantigen construct to be employed therapeutically will depend, for example, upon the therapeutic objectives, the route of administration, and the condition of the patient. In addition, the attending physician takes into consideration various factors known to modify the action of drugs including severity and type of disease, body weight, sex, diet, time and route of administration, other medications and other relevant clinical factors. Accordingly, it will be necessary for the therapist to titer the dosage and modify the route of administration as required to obtain the optimal therapeutic effect. Typically, the clinician will administer antibody/autoantigen construct until a dosage is reached that achieves the desired effect. The progress of this therapy is easily monitored by conventional assays.

For any antibody/autoantigen construct, the therapeutically effective dose can be estimated initially from cell culture assays. For example, a dose can be formulated in animal models to achieve a circulating concentration range that includes the EC₅₀ as determined in cell culture (e.g., the concentration of the test molecule which promotes or inhibits cellular proliferation or differentiation). Such information can be used to more accurately determine useful doses in humans.

Toxicity and therapeutic efficacy of the antibody/autoantigen constructs described herein can be determined by standard pharmaceutical procedures in cell cultures or experimental animals, *e.g.,* for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio between LD₅₀ and ED₅₀. Molecules which exhibit high therapeutic indices may be used. The data obtained from these cell culture assays and animal studies can be used in formulating a range of dosage for use in human. The dosage of such molecules may lie within a range of circulating concentrations that include the ED₅₀ with little or no toxicity. The dosage may vary within this range depending upon the dosage form employed and the route of administration utilized. The exact formulation, route of administration and dosage can be chosen by the individual physician in view of the patient's condition. (See *e.g.,* Fingl et al., 1975, in "The Pharmacological Basis of Therapeutics", Ch. 1 p.1.)

Dosage amount and interval may be adjusted individually to provide plasma levels of the antibody/autoantigen construct which are sufficient to promote or inhibit cellular proliferation or differentiation or minimal effective concentration (MEC). The MEC will vary for each antibody/autoantigen construct, but can be estimated from *in vitro* data using described assays. Dosages necessary to achieve the MEC will depend on individual characteristics and route of administration. However, HPLC assays or bioassays can be used to determine plasma concentrations.

Dosage intervals can also be determined using MEC value. Antibody/autoantigen construct molecules should be administered using a regimen which maintains plasma levels above the MEC for 10-90% of the time, preferably between 30-90% and most preferably between 50-90%.

In cases of local administration or selective uptake, the effective local concentration of the antibody/autoantigen construct may not be related to plasma concentration.

A typical daily dosage might range from about 1 µg/kg to up to 1000mg/kg or more, depending on the factors mentioned above. Typically, the clinician will administer the antibody/autoantigen construct until a dosage is reached that achieves the desired effect. The progress of this therapy is easily monitored by conventional assays.

Depending on the type and severity of the disease, from about 0.001 mg/kg to abut 1000 mg/kg, more preferably about 0.01 mg to 100 mg/kg, more preferably about 0.010 to 20 mg/kg of the antibody/autoantigen construct might be an initial candidate dosage for administration to the patient, whether, for example, by one or more separate administrations, or by continuous infusion. For repeated administrations over several days or longer, depending on the condition, the treatment is repeated until a desired suppression of disease symptoms occurs or the desired improvement in the patient's condition is achieved. However, other dosage regimes may also be useful.

The disclosed methods can be used to treat diabetes mellitus by inducing immune tolerance to insulin-producing β cells of the islets of Langerhans within the pancreas. Autoantigens of these cells are linked to antibodies which recognize the desired antigen-internalizing receptor. Suitable autoantigens for use in this disclosure are β cell antigens, and epitopes, or peptides representing epitopes, of insulin, glutamic acid decarboxylase ("GAD") and heat shock protein ("HSP"). Linking a set of peptides covering epitopes from insulin, GAD and hsp to an anti-DC-SIGNR antibody has the potential to induce tolerance to all major antigens implicated in T1 DM. Other autoantigens may be known and used, or discovered and used, by those skilled in the art.

The antigen-internalizing receptor is presented on specialized APCs. For this method, the antigen-internalizing receptor chosen is DC-SIGNR (dendritic cell-specific intercellular adhesion molecule 3-grabbing nonintergrin related receptor). DC-SIGNR is expressed by liver sinusoidal endothelial cells ("LSEC"), which are liver-resident antigen presenting cells. (Pohlmann et al, 2001). DC-SIGNR belongs to the family of pathogen internalization receptors that internalize receptor bound protein and facilitate antigen presentation. (¹Geijtenbeek et al., 2002). It has been shown that presentation of an antigen by LSECs results in an antigen- specific tolerance (Limmer et al., 2000). In contrast to other dendritic cell types that can mature from an immature tolerogenic state to an activating state, liver sinusoidal cells can not be induced to develop into an activating antigen presenting cell (²Knolle et al., 1999). The human DC-SIGNR (also called L-SIGN) homologue to human DC-SIGN shows 77% identity to DC-SIGN at the amino acid level and has the typical domain for internalizing receptors (Bashirova et al, 2001; Soilleux et al, 2000). DC-SIGNR is highly expressed on LSEC and is also found on a sub-population of lymph node macrophage-like cells, but is not expressed by DCs.

For purposes of the present disclosure, the terms "DC-SIGNR" and "L-SIGN" are used interchangeably.

The C-type lectin mouse DC-SIGN (CD209) has recently been identified as a DC-specific receptor. DC-SIGN mediates transendothelial migration of DCs, which enables primary immune responses by initiating transient DC-T cell interactions (³Geijtenbeek et al, 2000; ²Geijenbeek et al, 2000). DC-SIGN also serves as an internalizing antigen receptor recognizing pathogens through carbohydrate structures. Besides its prominent role in DC-T cell clustering and initiation of T cell responses, DC-SIGN is a major receptor involved in infection of DC and subsequent transmission to T cells of viruses such as HIV-1, HIV-2, SIV-1, hepatitis C virus (HCV), Ebola virus, cytomegalovirus (CMV), and Dengue virus; bacteria such as Helicobacter pylori, Klebsiella pneumonae, and Mycobacteria tuberculosis; yeast such as Candida albicans; and parasites such as Leishmania pifanoi and Schistosoma mansoni. The murine homologue of DC-SIGNR, mSIGNRI, captures antigens that are rapidly internalized and targeted for lysozomes for processing ('Geijtenbeek et al, 2002). Based on amino acid sequence, murine mSIGNR1 is equally homologous to human DC-SIGNR as it is to human DC-SIGN and is therefore useful for animal modeling studies.

The antibodies to DC-SIGNR may modulate, i.e. inhibit or enhance, the interaction of DC-SIGNR expressing cells with ICAM-expressing cells. The anti-DC-SIGNR antibodies may bind to the DC- SIGNR receptor site on the surface of an antigen presenting cell such as LSEC, and impede the interaction(s) between the LSEC and a T cell. More specifically, the antibodies to DC-SIGN reduce the adhesion between LSEC and T cells by interfering with the adhesion between DC-SIGNR and an ICAM receptor on the surface of a T cell.

As used herein, "ICAM receptor(s)" means both the ICAM-2 and ICAM-3 receptor, especially the ICAM-3 receptor.

The antibodies to DC-SIGNR of the present disclosure may not bind to DC-SIGN.

The antibodies to DC-SIGNR of the present disclosure may block entry of viruses into liver cells such as liver sinusoidal cells and their infection into other cells.

By interfering with the adhesion of T cells to antigen presenting cells, the use of antibodies to DC-SIGNR will affect antigen presenting cell-T cell clustering, T cell activation and other interactions that rely on contact between antigen presenting cells and T cells. These other interactions include both direct cell-to-cell contact or close proximity of antigen presenting cells and T cells.

In other embodiments, the anti-DC-SIGNR antibodies of the present disclosure are linked to peptides such as autoantigens, or self-antigen, peptides. These peptides can be linked to anti- DC-SIGNR antibodies by any suitable method, including grafting a vector to an antibody fragment and cloning the linked vector/antibody, or chemically linking. Methods of linking a vector, cloning or chemical linking are well known to those skilled in the art.

The peptides, preferably autoantigens, along with the linked antibody, are then internalized into the LSEC. LSECs bear surface molecules necessary for antigen presentation such as MHC II, CD80 and CD86 (Lohse et al, 1996; Rubinstein et al, 1986). In addition to inducing a regulatory phenotype in naive CD4+T cells (Knolle et al, 1999), LSECs can induce tolerance in CD8+ T cells by cross-presenting exogenous antigen (Limmer et al, 2000). LSECs respond to stimuli as TNF-α and endotoxin by downregulation of MHC, and hindering endosomal processing (²Knolle et al, 1999). Furthermore, LSECs do not migrate out of the liver to lymph organs.

This internalization facilitates the presentation of self-antigen peptides to the surface to the LSECs, mediated via MHC interactions. Once an autoantigen is expressed on the surface of the LSECs which have co-stimulatory potential, naïve autoreactive T cells can become activated. The T cells target and react with the linked autoantigen. The effector T cells kill few LSECs and die off without co-stimulatory molecules. This presentation of an autoantigen by LSEC results in autoantigen-specific tolerance.

The liver has a unique microenvironment with an abundance of tolerogenic mediators such as IL-10 and TGF-β and specialized APCs that favor the development of immunologic tolerance (¹Knolle & Gerken, 2000). Tolerogenic properties of the liver are supported by the finding that allogeneic liver transplants can be accepted across MHC barriers (Calne, 1969). Furthermore, application of antigens via the portal vein is more likely to lead to tolerance than systemic application of the antigen (Kamei et al, 1990). Draining through the liver has been reported to be a prerequisite for oral tolerance induction (Yang et al, 1994). Blood passing through the hepatic vessels first comes into contact with Kupffer cells and LSECs. The blood flow through the hepatic sinusoids is slow, allowing contact between the liver sinusoidal cell populations and passing leukocytes. LSECs bear surface molecules necessary for antigen presentation such as MHCII, CD80 and CD86 (Lohse et al, 1996; Rubinstein et al, 1986). In addition to inducing a regulatory phenotype in naive CD4+T cells (³Knolle et al, 1999), LSECs can induce tolerance in CD8+ T cells by cross-presenting exogenous antigen (Limmer et al, 2000). Klugewitz et al (Klugewitz et al, 2002) demonstrated that injection of Th1, IFN-γ producing TCR-transgenic cells into mice results after intravenous protein immunization in suppression of IFN-γ production by these cells in the liver and promotion of Th2-cells. In contrast to professional myeloid APC that can differentiate from an immature, tolerogenic stage into a mature stage initiating immunity, LSECs respond to stimuli as TNF-α and endotoxin by downregulation of MHC and hindering endosomal processing (²Knolle et al, 1999). Furthermore, LSECs do not migrate out of the liver to lymph organs. LSECs might not be the only APC specialized on inducing tolerance. Pugliese et al recently identified a small subset of spleen DCs that induced tolerance by presenting endogenously expressed autoantigen (Puglise et al, 2001). Overall, LSECs appear to be a favorable cell type for presenting β-cell antigens with the purpose of tolerance induction.

Practice of the described methods will be more fully understood from the following examples, which are presented for illustration only and should not be construed as limiting in any way.

### Example 1 - Obtaining anti-mSIGNR1 antibodies

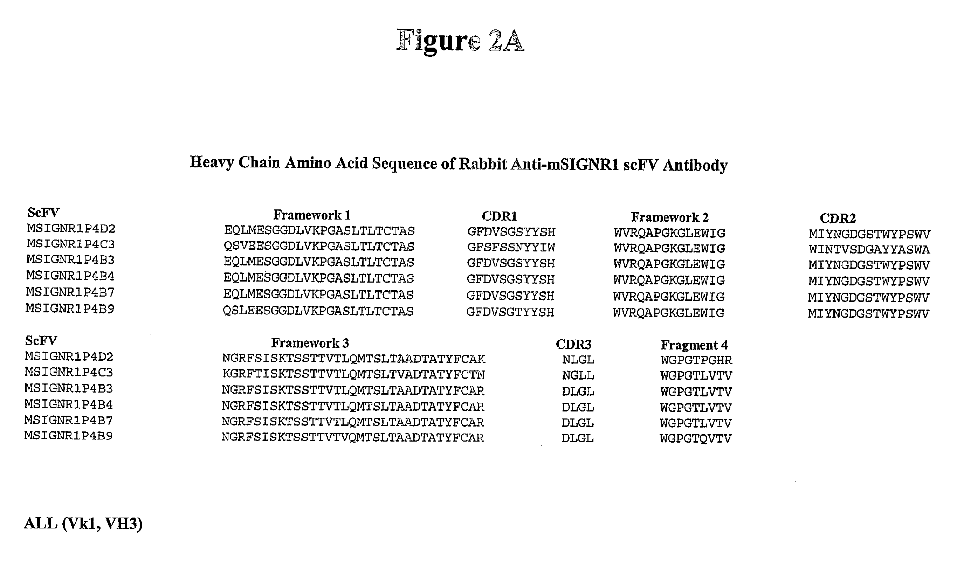

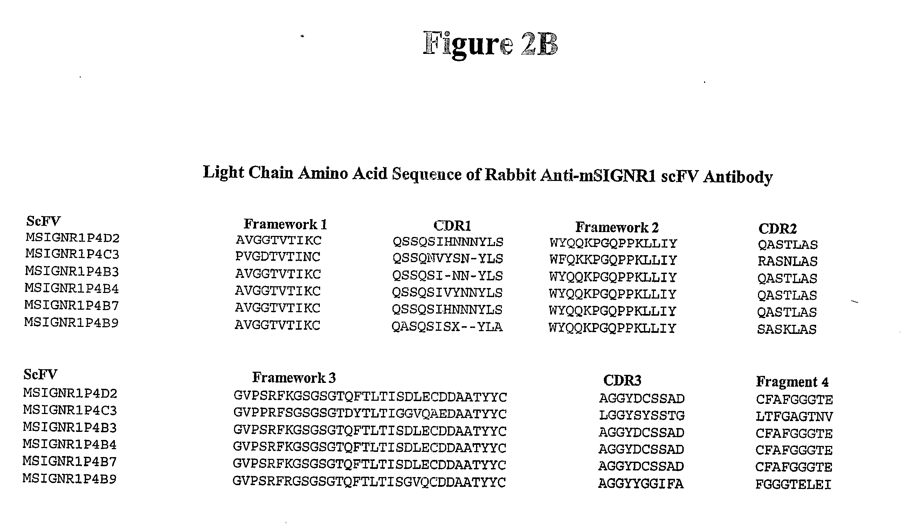

Using phage display technology, a panel of single chain antibodies (scFv) that recognize mSIGNR1 was identified. scFvs contain the variable light and heavy chain region connected by a linker. Their short length makes these antibody fragments very suitable for antigen linkage, and the capacity for binding to the receptor is preserved. Rabbits were immunized with recombinant mSIGNR1, and a scFv antibody library was constructed using the phage display vector pRL4 which is described in Published International Application No. WO 02/46436 A2 published on June 13, 2002. Antibody fragments in this system are displayed on the gene III coat protein of the phage. Antibodies recognizing mSIGNR1 were isolated by 4 rounds of solid phase panning on recombinant mSIGNR1. Six different antibodies were identified. The amino acid sequences of these six antibodies are presented in Figures 2A and B (SEQ. ID NOS: 1-6 and 7-12, respectively). All antibodies recognized mSIGNR1 in solid phase ELISA, and no cross-reactivity with mDC-SIGN, the murine homologue of human DC-SIGN, was observed. The antibodies were epitope-tagged with HA and HIS₆. Both mSIGNR1 and DC-SIGNHIS were produced by 3T3 EBNA cells and purified over a nickel column.

### Example 2 - Identifying anti-mSIGNR1 antibodies that are internalized upon binding to the cell surface receptor

### Screen for cell lines expressing mSIGNR1

A panel of murine macrophage cell lines (P388D1, 1-13.35, WEHI-3 and J774) are screened for expression of mSIGNR1 by RT-PCR by standard methods. Primers are designed based on the SIGNR1 Genbank sequence and used these in RT-PCR of mouse organs. A cell line expressing mSIGNR1 on the mRNA level is identified and surface expression is confirmed by FACS analysis. 5 x 10⁵ cells are incubated with 1 µg anti-mSIGNR1 antibody in PBS containing 1 % BSA and 0.1 % NaN₃ on ice for 15 minutes, conditions that do not allow for antibody internalization. After 2 washes with PBS containing 1 % BSA and 0.1 % NaN₃, bound anti-mSIGNR1 are detected by biotinylated anti-HA (Roche) followed by PE-conjugated streptavidin (Becton Dickenson) and cells are analyzed using FACS Calibur (Becton Dickinson). Alternatively, internalization is determined on primary cells known to express mSIGNR1 such as liver sinusoidal endothelial cells. Expression of mSIGNR1 on LSECs can also be confirmed by FACS as described above, but only 1 x 10⁵ cells is added per reaction.

### Measurement of internalization

Once a mSIGNR1-expressing cell line or primary cell type has been identified, internalization of the antibody panel is assessed by FACS analysis. To show that internalization is based on mSIGNR1 binding, a cell line that does not express mSIGNR1 such as JAWS1 mouse dendritic cells is included. Anti-mSIGNR1 detection using biotinylated anti-HA antibody followed by PE-conjugated steptavidin on intact and permeabilized cells is compared as described for anti-DEC-205 antibodies (Mahnke et al, 2000). I.5 x 10⁶ cells for cell lines or 3 x 10⁵ cells for primary cells are incubated with 3 µg of mSIGNR1 in PBS containing 1% bovine serum albumin (BSA) for 20 minutes at 4°C to allow for antibody binding to the surface without internalization. Unbound antibody is removed by washing 2 times with PBS containing 1 % BSA at 4° C. Each sample is divided into 3. One third is fixed with 4% paraformaldehyde and surface antibody is detected as described above. The other two thirds are further incubated for 30 minutes at 37°C to allow for internalization before being fixed. One half is directly detected with anti-HA and steptavidin, the other half is permeabilized by incubating the cells with PBS containing 0.1% (vol/wt) saponin (Sigma-Aldrich). The amount of internalized antibody is calculated by subtracting the mean fluorescence in fixed cells from that recorded with fixed and permeabilized cells. The antibodies with the highest percentages of internalization within 30 minutes are chosen for further studies linking peptides to the antibodies. An existing unrelated rabbit scFv is used as a negative control, the commercially available ER-TR9 antibody that has recently been shown to bind mSIGNR1 (¹Geijtenbeek et al, 2002) is used as a positive control. Also, Fab fragments of ER-TR9 are produced by papain digestion and tested for internalization to verify that dimerization is not a requirement for internalization. If desired, the scFvs can be converted into Fab'2 or IgG.

In an alternative embodiment, a mSIGNR1 library is panned for internalizing antibody as described by the group of James D. Marks (Poul et al., 2000). A suitable process for this embodiment is outlined below.

### Selection of internalizing antibodies from mSIGNR1 phage library

5 x 10⁶ cells identified as described above to express mSIGNR1 are incubated with 1 x 10¹² colony forming units of phage from a mSIGNR1 library presenting antibody fragments fused to gene 3 protein on their surface for 1.5 hours at 4°C to allow phage binding without internalization. After phage binding, the cells are washed 5 times with phosphate-buffered saline to remove non-specifically or weakly bound phage. Cells are then incubated for 15 minutes at 37°C to allow endocytosis of surface-bound phage, but avoid phage degradation within the cell. To remove phage bound to the surface of the cell, cells are stripped by washing three times with a low pH glycine buffer. Then cells are trypsinized and washed with PBS before being lysed with high pH triethylamine. The cell lysate containing phage are used to infect E. coli to prepare phage for the next round of selection. A total of three rounds of selection are performed. The titer of phage bound to the cell surface (found in the first low pH glycine wash) and the number of phage recovered from within the cell are monitored for each round. An increase in the number of endocytosed phage indicates a successful selection of internalizing phage antibody.

To determine whether any of the internalized scFv antibody fragments bind to mSIGNR1, 500 clones from round 3 are selected using a robotic Qpix (Genetix) system and grown in 96-well dishes in SB medium overnight in a HiGrow shaker (Gene Machines). The next day, dishes are spun down and supernatants tested in solid phase mSIGNR1 ELISA using a robotic Genesis freedom 200 (Tecan) system. 96-well ELISA plates are coated with 1 µg mSIGNR1/ml PBS overnight at 4°C. The next day, plates are blocked by 1% BSA, followed by 3 washes with PBS containing 0.05% Tween. Control plates are coated with 1% BSA. Supernatants containing antibody are added to mSIGNR1 or BSA alone wells at concentrations between 0.05 - 5µg/ml in PBS containing 1% BSA. After 2 hours on a shaker at room temperature, plates are washed 3 times with PBS containing 0.05% Tween. For detection of bound scFv, anti-HA antibody (12CA5 mouse ascites, Strategic Biosolutions, DE) are added at a 1:I,000 dilution in PBS with 1% BSA. After 2 hours on a shaker at room temperature, plates are washed again and alkaline-phosphatase-conjugated antimouse IgG (Sigma) are added for 2 hours. After 3 more washes, bound antibody are detected using Sigma 104® substrate. The plates are read at various time points at OD₄₀₅ with an ELSA plate reader (Molecular Devices).

Clones giving a positive signal in ELISA are characterized by restriction enzyme digest pattern. DNA are isolated using Qiagen's miniprep kit. 2µg of DNA are digested with 5 U of EcoRII for 2 hours at 37°C and then the samples are run on a 4% NuSieve agarose gel. Patterns are compared and sequences are purified in small quantities (about 100 - 300 µg). scFvs are properly assembled in the periplasmic space of bacteria and are secreted. scFvs can either be isolated from the supernatant or the periplasmic space. Clones are grown in 4 liter of SB to an OD₆₀₀ of 0.8 and induced with 1 mM isopropyl-p-D-thiogalactopyranoside (IPTG) for 3-4 hours at 30°C to produce optimum amounts of scFv. To isolate single chain antibodies form the periplasmic space, cell pellets are resuspended in cold PBS with added Complete Mini (Roche) protease inhibitor and are sonicated using a Sonics Vibra-cell VC750. Cellular debris is then pelleted and the supernatants are applied to Qiagen Ni-NTA columns using an Akta FPLC (Pharmacia). Antibody is eluted with imidazole. This method generally yields about 100-300 µg of purified antibody/liter. Endotoxin is removed by filtration through Sartorius QI5 filters generally yielding antibody preparations containing less than 10 U/ml endotoxin as determined by LAL test (an assay commercially available from Bio Whittaker). Antibodies are analyzed again for internalization as described above as well as for binding to recombinant mSIGNR1 in solid phase ELISA. The antibody with the highest percentage of internalization within 30 minutes and a good signal in a solid phase ELISA (> 10D after 1 hour at 1 µg/ml) are selected to make peptide-antibody constructs.

### Example 3 - Link GAD peptides to the antibody

### Vector and cloning strategy

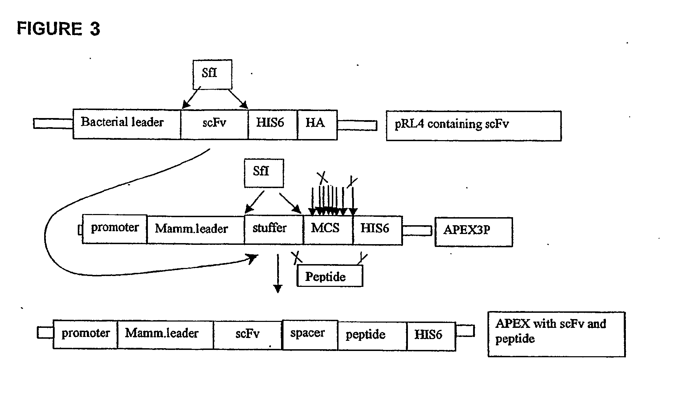

Following identification of the best bacterially-produced scFv, a conversion to a mammalian expression system is made. Mammalian expression allows for the appropriate secondary modifications of the peptides and endotoxin-free production. A vector (e.g., described in U.S. Patent No. 6,355,245) with compatible restriction sites as shown in figure 3 is used. DNA from the antibody of interest in pRL4 (described above) are cut with Sfi and inserted into the Apex 3P vector containing a CMV promoter and mammalian antibody leader sequence. To insert nucleotides encoding the peptides of interest, restriction sites are chosen from the sites available (MCS=Nael, Fsel, Xbal, EcoRI, Pstl, EcoRV, BSABI, BstXI, Notl, BsrBI, Xho, PbvIOI, Sphl, Nsil, Xbal) that are not contained within the antibody sequence. Oligonucleotides encoding peptides are synthesized by Operon with the appropriate restriction sites at each end and are inserted using T4 DNA ligase. The resulting construct will contain the scFv followed by a spacer determined by the restriction enzyme chosen followed by a peptide and a HIS-tag (Figure 3). Sequences are confirmed using standard techniques before transfecting DNA into 393EBNA cells for antibody production.

### Choice of peptide

While it should be understood that any of the peptides of the known diabetes autoantigens insulin, hsp and GAD 65 and 67 can be used in the processes described herein, for the following experiment GAD is chosen as the peptide. GAD-reactive T cells are the first autoreactive T cells to be detected in the NOD mouse (Tisch et all 1993; Kaufman et al, 1993) and have been shown to be important in the disease process. Furthermore, human and murine GAD are 95% homologous. Epitopes recognized by splenic NOD T cells have been extensively characterized (Kaufman et al, 1993; Tisch et all 1999; Zechel et al. 1998) and many immunodominant peptides are similar in NOD mice and T1 DM patients and have been used interchangeably in in vitro T cell assays (Kaufman et all 1993). The initial immune response in NOD mice is directed against a defined region in the carboxy-terminal region of GAD65 (peptide 509-528, peptide 524-543 (Kaufman et all 1993). Later T cell responses are also directed against other regions between 200-300 as well as other autoantigens. One of the early CD4 GAD65 T cell epitopes, peptide 524-543 (SRLSKVAPVIKARMMEYGGT (SEQ. ID NO: 13), same sequence in mice and humans) and two of the later occurring murine GAD65 epitopes, peptide 247-266 (NMYAMLIARYKMFPEVKEKG (SEQ. ID NO: 14), 1 amino acid difference between human and mouse underlined), and peptide 290-309 (ALGIGTDSVILIKCDERGK (SEQ. ID NO: 15), same sequence in mice and humans) are selected for use as the peptides in this experiment. All 3 epitopes can induce a spontaneous proliferative response in NOD splenocytes. Furthermore, peptide immunization with peptide 247-266 and peptide 290-309 have been shown to delay diabetes onset in NOD mice (Ma et all 1997; Tisch et all 1999; Zechel et all 1998). In addition to CD4 T cell epitopes, tolerance to CD8 T cell epitopes has also been reported to be important (Quinn et all 2001; Bercovici et al, 2000). As a negative control, an antibody construct is made with hen egg lysozyme peptide 116-1 24 (KGTDVQAWI) (SEQ. ID NO: 16). The most effective peptides from these in vitro studies are then linked in various combinations with an antibody construct and tested in the NOD diabetes model.

### Example 4 - Antibody-peptide construct production and purification

For T cell experiments, approximately 300 µg of each antibody construct is produced in EBNA293 human embryonic kidney cells. Cells are grown in DMEM with 10% FCS, 2 mM glutamine and 250 U/ml G418 (Sigma). Cells in TI75 flasks are transfected with DNA using Qiagen's Effectine reagent according to the manufacturer's instruction. Medium is exchanged for serum-free medium after 3 days. Supernatant is collected at day 4 and day 8, cell debris is removed by centrifugation and the cleared supernatant is loaded on a Ni-column using a Akta-FPLC. Antibody is eluted with imidazole, dialyzed into PBS and correct size verified by running 1 µg on a SDS- gel.

### Example 5 - Internalization of the antibody-peptide construct by LSEC resulting in peptide presentation and the effect of this presentation on T cells

Liver sinusoidal cells are targeted in vitro with the peptide-antibody construct and it is determined whether these cells can induce a phenotypic change in T cells derived from young NOD or Balb Ic mice.

### Isolation of murine sinusoidal endothelial cells

Liver sinusoidal endothelial cells are isolated from 3 week old NOD or 4-6 week-old Balb/c mice. Cells are obtained by portal perfusion first with EGTA to chelate calcium and loosen cell-cell contacts followed by perfusion with 0.05% collagenase A in Hank's buffer to degrade intercellular matrix as described by Kretz-Rommel (Kretz-Rommel & Boelsterli, 1995). The perfused liver is removed from the mouse and gently worked with a pair of angled forceps. The resulting crude cell suspension is filtered through a series of metal sieves (30, 50, 80 mesh) to remove larger tissue fragments. Sinusoidal cells are separated from parenchymal cells by density gradient centrifugation on a metrizamide gradient (1.089 g/cm3) followed by 2 washing steps to remove cell debris (3Knolle et al, 1999). At this point, a mixture of Kupffer cells and liver sinusoidal cells is obtained. For FACS experiments this is sufficient, since Kupffer cells can be distinguished from liver sinusoidal endothelial cells using the F4/80 antibody that recognizes Kupffer cells, but not liver sinusoidal cells. However, for co-culture experiments with T cells and peptide, Kupffer cells are removed by labeling the cells with PE-conjugated F4/80 (BD Pharmingen) followed by Miltenyi's anti-PE microbeads and magnetic sorting of the labeled cells using MACS column and separator according to the manufacturer's instructions. The remaining cell population is seeded onto 96 well tissue culture plates in Dulbecco's modified Eagle medium (DMEM) supplemented with 10% fetal bovine serum and 2% glutamine. The purity of cell populations is investigated at day 3 after isolation by FACS staining for surface markers using anti-mSIGNR1 and anti-F4/80. mSIGNR1 is absent on Kupffer cells (¹Geijtenbeek et al, 2002). 90% purity is considered sufficient to proceed with the experiments. 2 mouse livers are used per experiment with an expected yield of about 2 x 10⁷ cells (Knook & Sleyster, 1976, extrapolated for mouse).

### T cell phenotypic assays

T cell assays demonstrate if the peptide-antibody construct results in presentation of peptide by liver sinusoidal endothelial cells and whether peptide presentation can induce a phenotypic change in T cells. Liver sinusoidal endothelial cells are cultured in flat bottom microtiter plates at a density of 1 x 10⁵ cells/well. After maintaining the sinusoidal cells for 3 days, CD4+ T cells will be purified as described above from a 3-week old and an 8-week old NOD mouse or a 4-6 week-old Balb/c mouse and are added at 10⁴ or 10⁵ cells/well. Also, each antibody-peptide construct at concentrations of 0.1 -5 pg/well is added. As a positive control, each GAD and control peptide by itself are included. Peptides are synthesized by SynPep (Dublin, CA). Negative control wells include either T cells alone or liver sinusoidal cells alone.

There are 4 possible outcomes of peptide presentation by LSECs to T cells: 1) induction of regulatory T cells characterized by the production of TGF-P and/or IL-10 and IL-4 or the expression of CD4+CD25+CD62L, 2) deletion of T cells or 3) a complete lack or response. 4) It is also conceivable that peptide presentation instead of inducing tolerance results in stimulation of Th1 cells producing IL-2. To distinguish among these possibilities, culture supernatants (100 pl each) are collected at 24 and 48 hours and assayed for cytokine production as described below. T cell responses of 3-week old are compared with those of 8-week old NOD mice as well as with T cells derived from Balb/c mice that do not show a spontaneous response to GAD. Assays are set up in triplicate and repeated twice. Since a mixture of T cells is used, a cytokine response in the supernatant might not be easily seen. However, a mixture of cells reflects the situation in vivo and GAD-specific T cell responses are seen using total splenocytes (Tisch et al, 1993).

As a more sensitive measure for the induction of regulatory T cells, cells are evaluated for the expression of typical surface markers such as CD25, CD4 and CD62L (Lafaille & Lafaille, 2002) by FACS analysis after 3 days in culture. All reagents are available from BD-Pharmingen. Also, IL-4 production is analyzed by FACS. A functional test of potential immunoregulatory properties of the LSEC/peptide exposed T cells as described below are the ultimate test for tolerance induction in this system. The possibility of peptide presented by LSEC inducing cell death are assessed in culture supernatants using Roche's cell death ELISA kit according to the manufacturer's instructions.

### Isolation of splenocytes and CD4+ T cells

Spleens from NOD of Balb/c mice are removed in a sterile environment and put in PBS as routinely performed in our laboratory. Cells are separated using 18-21 gauge needles, and larger pieces are allowed to settle. Supernatant is removed and centrifuged at 200 g for 7 min. Red blood cells are lysed using 5 ml 0.83% NH₄Cl per spleen. Cells are washed twice in PBS and then resuspended in medium. For certain experiments, total splenocytes are used. For other experiments, CD4+ T cells are isolated using Miltenyi's (Auburn, CA) CD4+ T cell isolation kit according to the manufacturer's instruction. Magnetic isolation of various cell populations is within the purview of one skilled in the art. In one process, isolation is based on depletion of non-CD4+ T cells using a cocktail of biotin- conjugated monoclonal antibodies against CD8a, CDI 1 b, CD45R, DX5 and Ter- 1 19. The purity of the isolated population is assessed by staining of a mixture of FITC-conjugated anti-CD4, PE-conjugated anti-CD8, APC-conjugated anti-CD11 b and cy-5-conjugated CD45R (all eBioscience, San Diego, CA). Expected purity is 90-95% with 70% yield. Since at least 1 x 10⁸ cells can be obtained from a mouse spleen and about 25% of splenocytes are CD4+, about 1.75 x 10⁷ cells can be obtained, enough for 175 96-well microtiter plate wells.

### Measurement of cytokine production

Presence of IL-10, TGF-P, IFN-γ, IL-4 and IL-2 in supernatants of T cell/LSEC co-cultures are determined by standard sandwich ELISA as described (Kretz-Rommel & Rubin, 1997). All antibody pairs are available from BD Pharmingen. A cytokine capture antibody is coated on the plate in PBS overnight at 4°C. After 3 washes with PBS/0.05% Tween, culture supernatants and a standard curve of mouse recombinant cytokine are added and incubated for 2 hours on a shaker at room temperature. Plates are washed again and bound cytokine is detected with an alkaline-phosphatase conjugated anti-cytokine antibody. After 3 washes, Sigma 104^{™} substrate is added and the plates are read at various timepoints at OD₄₀₅ with an ELISA plate reader (Molecular Devices).

### Example 6 - Assess whether T cells exposed to GAD peptides presented on LSECs can subsequently prevent activation of autoreactive T cells by professional APCs presenting GAD

In a further experiment, whether T cells exposed to peptides on liver sinusoidal endothelial cells for 3 days can negatively regulate activation of autoreactive T cells by peptide presented on splenic professional APCs is tested. Feasibility of the induction of T cells with regulatory properties in vitro has been demonstrated by a number of laboratories (Wakkach et all 2001 ; Barrat et all 2002; Thorton & Shevach, 1998). Immunosuppressive properties can be tested by adding the regulatory T cells to a culture system in which immune stimulation is normally observed. Addition of GAD peptide to spleen cells from a 7 week-old NOD mouse comprising both APCs and T cells provides such an immunostimulatory system as seen by a strong proliferative response. Addition of regulatory T cells abrogates this response. 10⁵ or 10⁶ splenocytes are added together with either 0.1, 1, or 10 µm peptide per well containing T cells exposed to LSEC and peptide-coupled antibody. Control wells include splenocytes with peptide alone and LSEC + T cells alone. Furthermore, to exclude significant contribution of proliferation by the presumed regulatory T cells, control wells also contain irradiated splenocytes (600 RAD, performed at UCSD irradiation service facility by Joe Aguilera) and T cells previously exposed to LSEC and the peptide-antibody construct. Irradiated splenocytes can present antigen, but do not proliferate. 1µCi ³H-thymidine is added to each well during the last 16 hours of a 72 hour culture period to label newly synthesized DNA as a readout for proliferation. Cells are harvested using Packard's Universal Cell Harvester and incorporated ³H-thymidine is assessed using a Topcount (Packard). If ³H-thymidine incorporation in cultures containing splenocytes and T cells previously exposed to LSEC+peptide is reduced compared to the splenocyte cultures, T cells have been successfully induced with regulatory properties. Whether the peptide-conjugated anti-mSIGNR1 antibody can induce regulatory T cells in the NOD mouse, and whether disease can be halted are also tested.

### Example 7

Mouse anti human L-SIGN antibodies were identified using recombinant phage technology. Mouse libraries (IgG1k and IgG2ak) derived from heavy and light chain combination of mice immunized with recombinant human L-SIGN were prepared by the methods disclosed in WO 03/025202. Once prepared, the libraries were first panned on human DC-SIGN to remove antibodies cross reactive with DC-SIGN. The unbound supernatants were used for selecting clones uniquely reactive with L-SIGN. A total of ninety-five colonies (36/round) for each of the two libraries (IgG1 & IgG2a) were induced and antibody production and their reactivity with L-SIGN were determined by a capture ELISA. Briefly, anti-human Fc (Caltag) was coated on ELISA. plates at 500 ng/ml overnight. The plates were blocked with PBS containing 1% BSA followed by the addition of recombinant L-SIGN at 2 µg/ml. After washing the plate with PBS, supernatants were added. After a 12 hour incubation at room temperature, plates were washed 3 times and an alkaline-phosphatase-conjugated anti-Fab antibody was added for 2 hours. Signal after addition of SigmaS substrate was assessed using an ELISA reader (Molecular Devices).

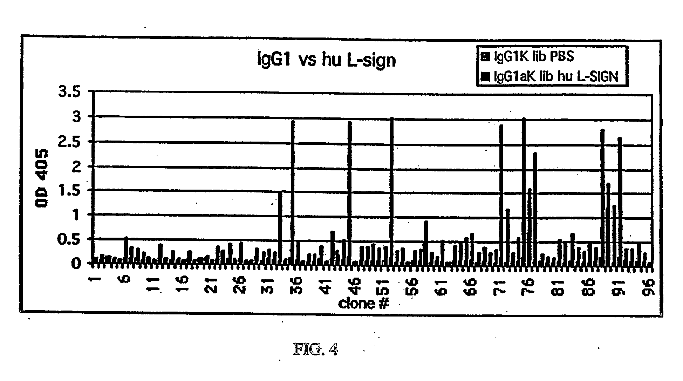

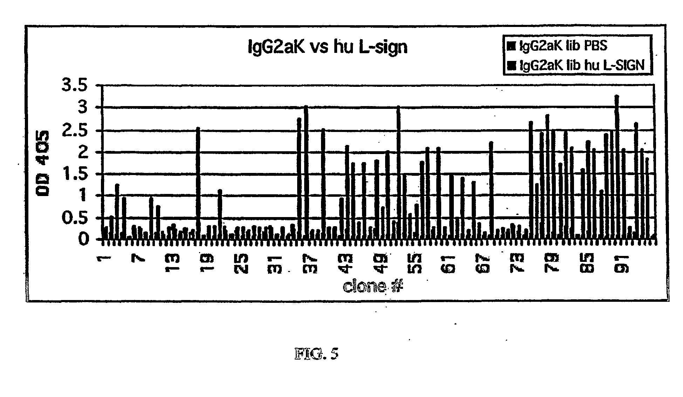

The majority of the clones showed good binding (OD405 >1.0) of the antibody on the phage. Several clones from both IgG1 and IgG2a libraries showed positive reactivity with human L-SIGN. Results are set forth in Figures 4 and 5: Figure 4 sets forth the IgG1 clones with human L-SIGN; Figure 5 sets forth the reactivity of IgG2a clones with human L-SIGN.

### Example 8

To identify clones uniquely reactive with human L-SIGN, all clones from Example 7 with OD values of five-fold above background were selected to test their reactivity with human DC-SIGN by ELISA. Anti-human Fc (Caltag) was coated on ELISA plates at 500 ng/ml overnight. The plates were blocked with PBS containing 1% BSA followed by the addition of recombinant DC-SIGN at 2 µg/ml. After washing the plate with PBS, supernatants were added. After a 12 hour incubation at room temperature, plates were washed 3 times and an alkaline-phosphatase-conjugated anti-Fab antibody was added for 2 hours. Signal after addition of SigmaS substrate was assessed using an ELISA reader (Molecular Devices).

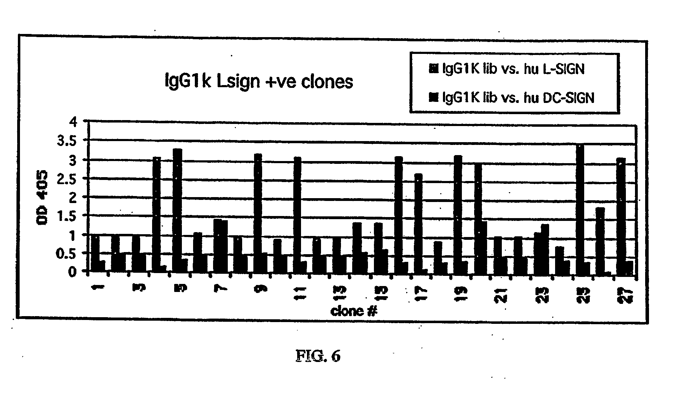

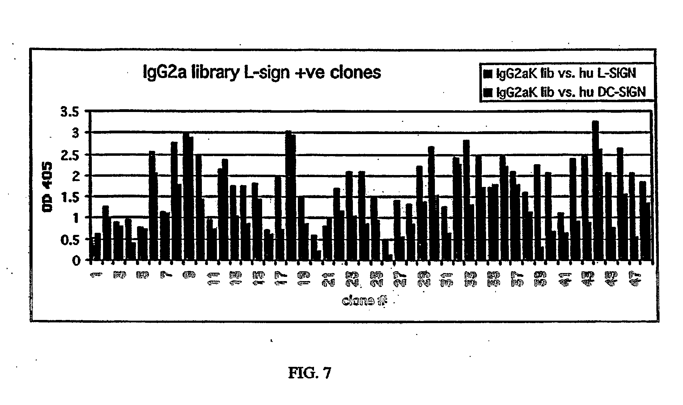

Ten clones from the IgG1 library and three clones from the IgG2a library were found to uniquely react with human L-SIGN (five to ten fold higher OD values vs. DC-SIGN). Results are set forth in Figures 6 and 7: Figure 6 sets forth the reactivity of the IgG1 positive clones with L-SIGN and DC-SIGN; Figure 7 sets forth the reactivity of the IgG2a clones with L-SIGN and DC-SIGN.

### Example 9

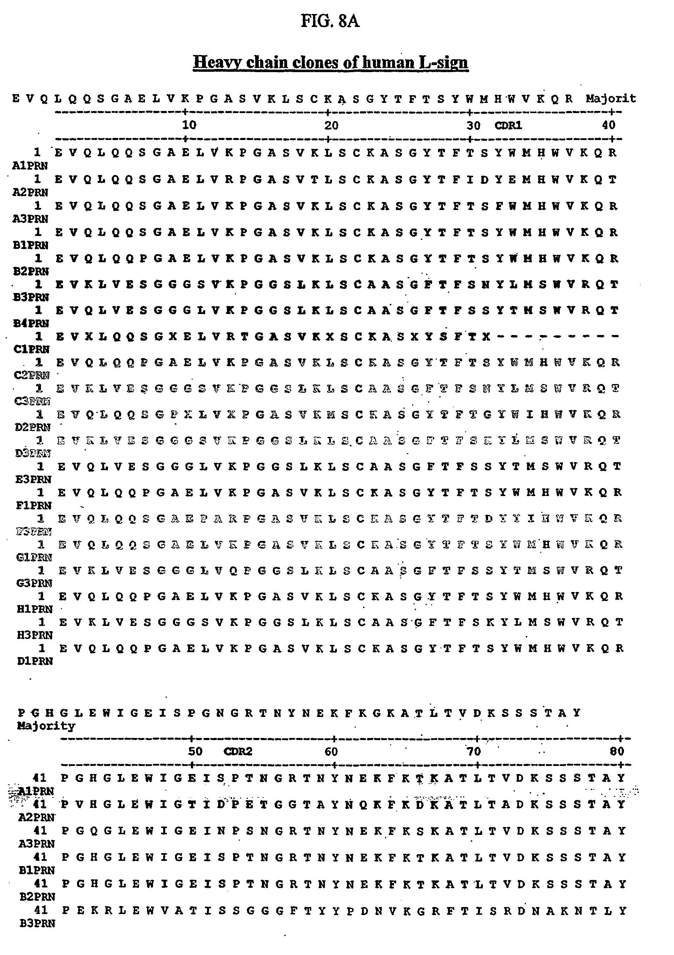

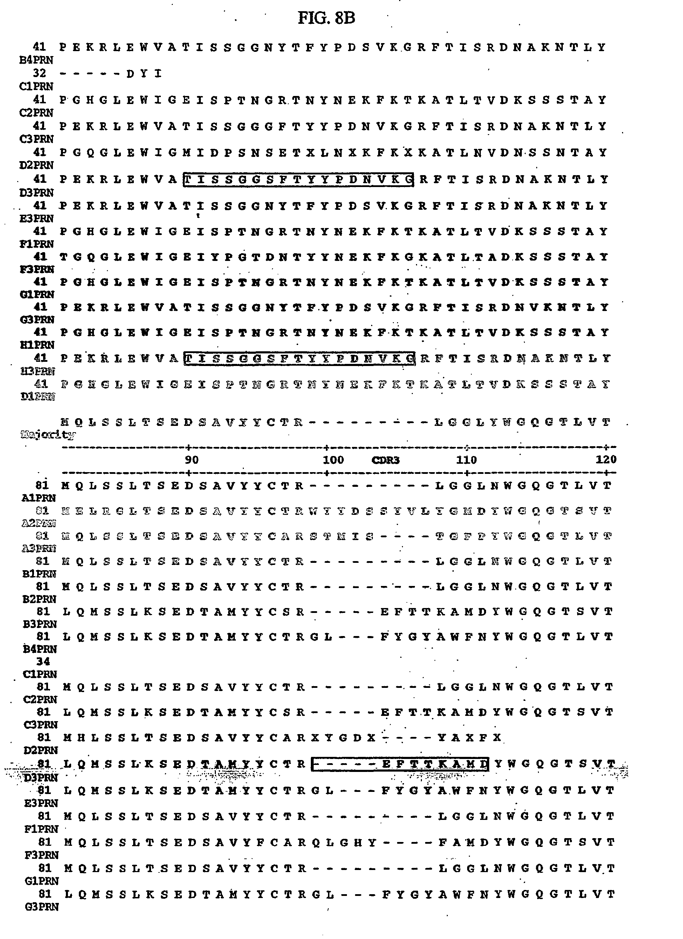

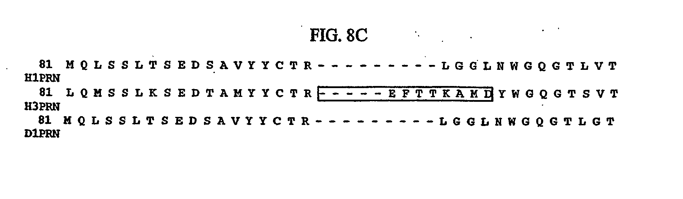

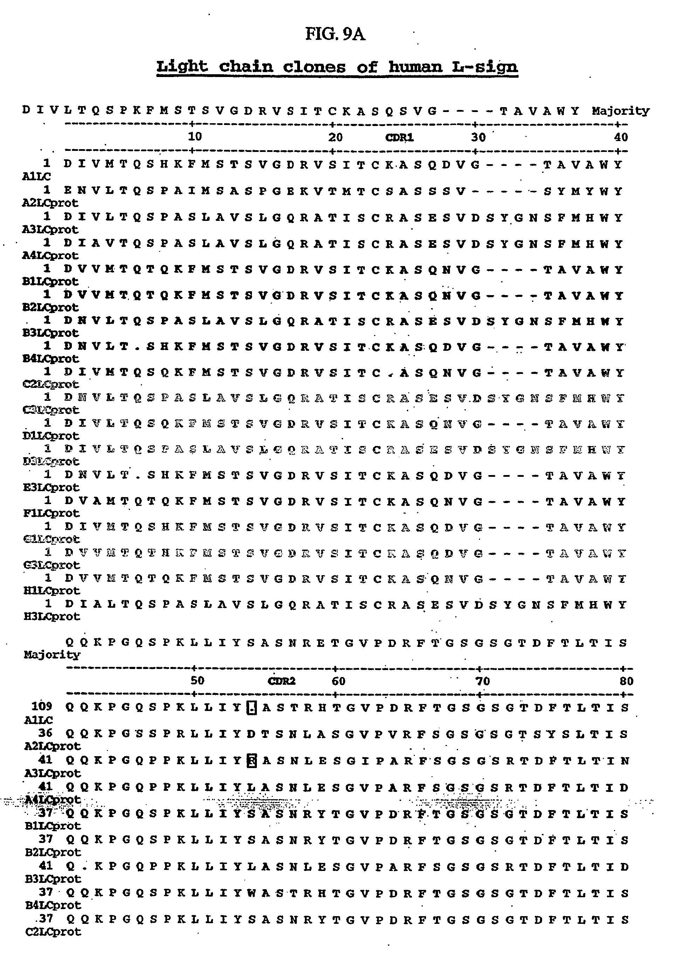

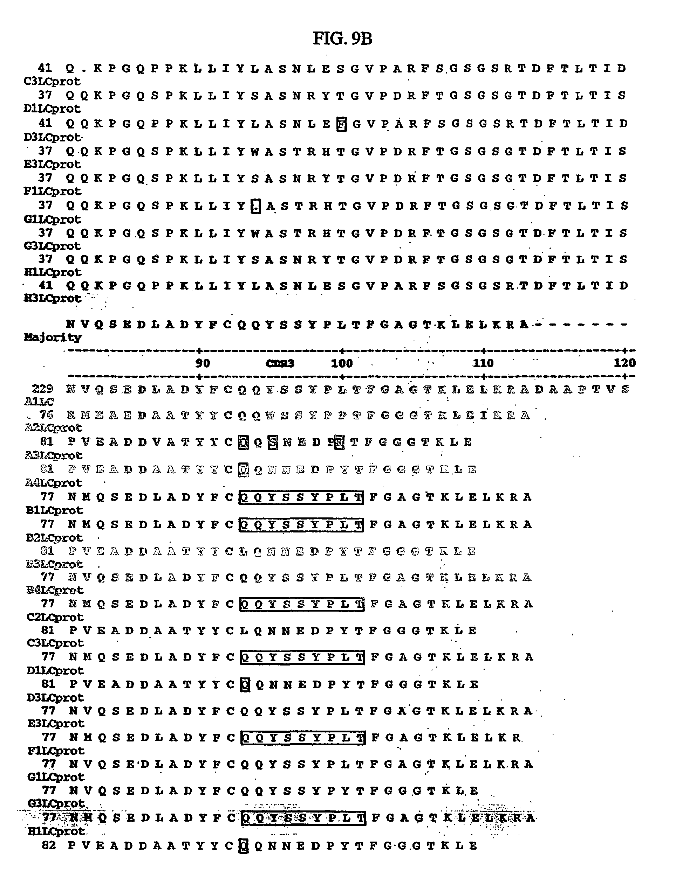

Thirteen clones identified as reactive with only human L-SIGN and nine clones strongly reactive with both L-SIGN and DC-SIGN as identified above in Example 8 were sequenced to determine the number of unique clones. Sequencing was determined by techniques known to those skilled in the art. The sequences of these clones are set forth in Figures 8 and 9; sequences for heavy chain clones are set forth in Figure 8A-8C (SEQ. ID NOS. 17-36); sequences for light chain clones are set forth in Figures 9A-9B (SEQ. ID NOS: 37-55).

Sequencing results demonstrated a more diverse group of heavy chains compared with light chains. The diversity of the antibody clones was also enhanced by cross pairing of different light chains with the same heavy chain. A total of five unique clones reactive with L-SIGN were identified and clones were grouped based upon the similarity of their amino acid sequences (see Table 1 below).

**TABLE 1**

| Grouping of antibody clones reactive with human L-SIGN based on amino acid sequence similarities | | | |
|---|---|---|---|
| Ab clusters | Light chain | Heavy chain | Unique L-sign clones |
| 1 | B1;B2;D1;F1;H1 | 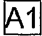 ^{a}; 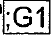 ^{a}; B1 (identical) | 2 |
| | C2 (stop/CDR1) | B2;C2;D1;F1;H1 (1/FR1 vs. A1, B1, G1) | |
| 2.1^{c} | B3;C3 | B3 ;C3^{a} | 1 |
| 2.2 | D3(1/CDR2); H3 | D3;H3^{b} | 0 |
| 2.3 | A3 (1/CDR2); A4^{b} (1/CDR3) | A3^{a} | 1 |
| 3.1 | B4;E3 (stop/FR1) | B4^{b}, E3^{b} | 0 |
| | G3 (1/CDR3) | G3^{b} (2/FR1;1/FR2) | |
| 3.2 | 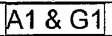 (stop/CDR2) | | |
| 4 | | D2^{a} | 1 |
| 5 | A2 | A2^{b} | 0 |
| 6 | | F3^{b} | 0 |
| 7 | | C1^{b} (incomplete seq) | 0 |

| | | | |
|---|---|---|---|
| ^{a}Antibody clones reactive with only L-SIGN ^{b}Antibody clones reactive with both L- and DO-SIGN ^{c} 2.1...2.3 designates similar light chains but unique heavy chains | | | |

The reactivities (OD values) of the clones selected for sequencing with L-SIGN and DC-SIGN are set forth below in Table 2.

**TABLE 2**

| IgG1k library | | | IgG1k library | IgG2ak library | | | IgG2ak library |
|---|---|---|---|---|---|---|---|
| Seq. Well # | deep well# | OD405 | clones to purify | Seq. Well # | deep well# | OD405 | clones to purify |
| A1 | A5 | 3 | A5 | A3 | E11 | 2.2 | E11 |
| B1 | C5 | 3.2 | | B3 | F11 | 2 | |
| C1 | B6* | 1.4 | | C3 | F12 | 2 | F12 |
| D1 | E6 | 3.2 | | D3 | A3* | 2 | |
| E1 | D7 | 3.1 | | E3 | D5* | 2.9 | |
| F1 | G9 | 3.1 | | F3 | C6* | 2.3 | |
| G1 | H9 | 2.6 | | G3 | D7* | 2.9 | D7 |
| H1 | C10 | 3.1 | | H3 | E10* | 2.2 | |
| A2 | C11* | 1.4 | | A4 | A11* | 2.2 | |
| B2 | H11 | 3.5 | H11 | B4 | B12* | 2.6 | |
| C2 | B12 | 1.8 | | | | | |
| D2 | C12 | 3.1 | C12 | | | | |

| | | | | | | | |
|---|---|---|---|---|---|---|---|
| * = Those clones selected for sequencing with L-SIGN and DOC-SIGN | | | | | | | |

As set forth in Figure 8, heavy chain CDR3 regions of the antibodies that bind to human DC-SIGNR were found have one of the following amino acid sequences: LGGL (SEQ. ID NO: 56); EFTTKAMD (SEQ. ID NO: 57); GLFYGYAWFN (SEQ. ID NO: 58). As set forth in Figure 9, light chain CDR3 regions of the antibodies that bind to human DC-SIGNR were found to have one of the following amino acid sequences: QQYSSYPLT (SEQ. ID NO:59); QQSNEDPRT (SEQ. ID NO: 60); QQNNEDPYT (SEQ. ID NO: 61); LQNNEDPYT (SEQ. ID NO: 62).

### Example 10

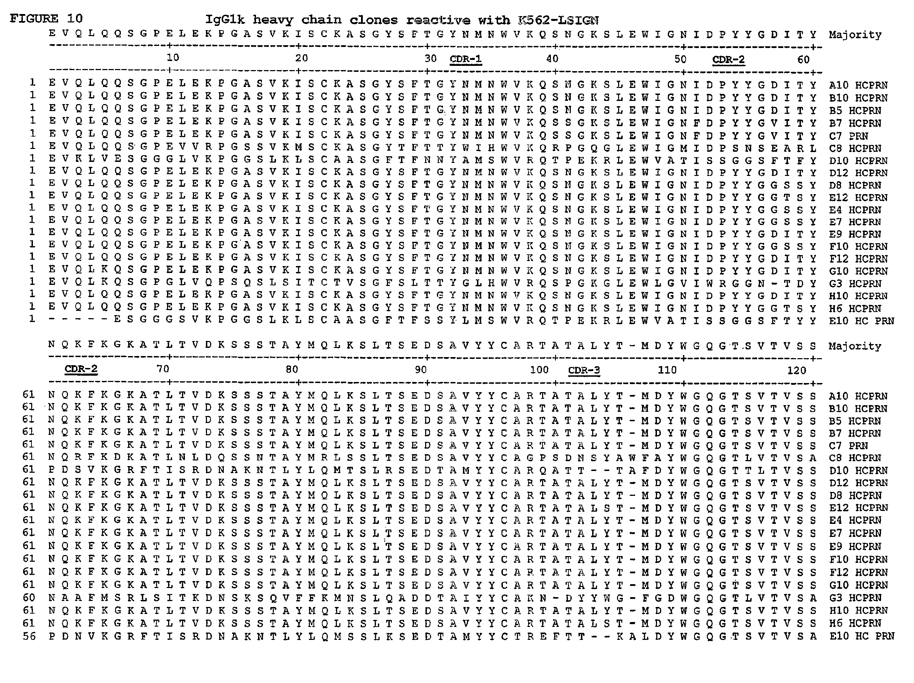

Additional clones from Example 7 above were examined for their ability to bind to L-SIGN utilizing the procedures described above in Example 7. Sequencing was determined by techniques known to those skilled in the art. The sequences for these additional clones are set forth in Figure 10 (set. ID NOS: 63-82). As set forth in Figure 10, five additional heavy chain CDR3 regions of the antibodies that bind to human DC-SIGNR were found have one of the following amino acid sequences: PSDNSYAWFA (SEQ. ID NO: 83); QATTTAFD (SEQ. ID NO: 84); TATALSTMD (SEQ. ID NO: 85); NDYYWGFG (set. ID NO: 86); TATALYTMD (SEQ. ID NO: 87); and EFTTKALD (SEQ. ID NO: 88). The CDR2 regions of these clones that bound to human DC-SIGNR were found to have one of the following amino acid sequences: MIDPSNSEARLNQRFKD (SEQ. ID NO: 89); TISSGGSFTFYPDSVKG (SEQ. ID NO: 90); NIDPYYGGTSYNQKFKG (SEQ. ID NO: 91); VIWRGGNTDYNAAFMS (SEQ. ID NO: 92); NFDPYYGVITYNQKFKG (SEQ. ID NO: 93); NIDPYYGGSSYNQKFKG (SEQ. ID NO: 94); and TISSGGSFTYYPDNVKG (SEQ. ID NO: 95).

Table 3 shows additional IgG1k antibody clones selected based on their reactivity with cells expressing L-SIGN

**TABLE 3**

| **Ab clusters** | **Light chain** | **Heavy chain** | **Unique clones** |
|---|---|---|---|
| 1 | A10, H10 | A10, B10, B5, D12, E9, F12, G10, H10 | A10, H10 |
| 2 | D8, F10 | D8, E4, E7, F10 | D8, F10 |
| 3 | E12, H6 | E12, H6 | E12, H6 |
| 4 | B7, C7 | B7, C7 | B7, C7 |
| 5 | C8 | C8 | C8 (stop/LC) |
| 6 | D10 | D10 | D10 |
| 7 | E10 | E10 | E10 |
| 8 | G3 | G3 | G3 (stop/LC) |
| 9 | B5 | - | B5 (stop/LC) |
| 10 | B10 | - | B10 |
| 11 | D12 | - | D12 |
| 12 | E4 | - | E4 |
| 13 | E7 | - | E7 |
| 14 | E9 | - | E9 |
| 15 | F12 | - | F12 |
| 16 | G10 | - | G10 |

Table 4 shows the reactivity of additional IgG1k antibody clones with cells expressing LSIGN (Geometric Mean fluorescence) and recombinant L-SIGN and DC-SIGN proteins (OD values)

**TABLE 4**

| **IgG1k** | **Geo. Mean Flourecence** | **Geo. Mean Flourecence** | **OD405** | **OD405** |
|---|---|---|---|---|
| Clone Name | K562 | K562/L-SIGN | L-SIGN | DC-SIGN |
| A10 | 3.4 | 21.3 | 2.9 | 0.1 |
| B5 | 1.5 | 10.5 | 2.1 | 0.1 |
| B7 | 2.2 | 90.9 | 2.7 | 0.1 |
| B10 | 2.0 | 53.1 | 3.5 | 0.1 |
| C7 | 2.0 | 101.0 | 3.5 | 0.1 |
| C8 | 1.7 | 13.4 | 0.1 | 0.1 |
| D8 | 1.8 | 17.3 | 2.1 | 0.1 |
| D10 | 1.6 | 10.2 | 0.9 | 0.5 |
| D12 | 2.0 | 152.0 | 3.5 | 0.1 |
| E4 | 1.9 | 50.4 | 3.5 | 0.1 |
| E7 | 1.8 | 19.3 | 1.4 | 0.1 |
| E9 | 1.9 | 25.5 | 2.9 | 0.1 |
| E10 | 1.7 | 27.2 | 2.6 | 0.6 |
| E12 | 3.0 | 22.9 | 2.9 | 0.1 |
| F10 | 2.4 | 13.8 | 0.8 | 0.1 |
| F12 | 2.8 | 168.7 | 3.5 | 0.1 |
| G1 | 2.1 | 14.1 | 2.0 | 0.1 |
| G3 | 1.6 | 41.5 | 0.4 | 0.1 |
| G10 | 2.0 | 86.1 | 2.0 | 0.1 |
| H6 | 2.4 | 26.2 | 3.5 | 0.1 |
| H10 | 3.4 | 12.3 | 2.7 | 0.1 |

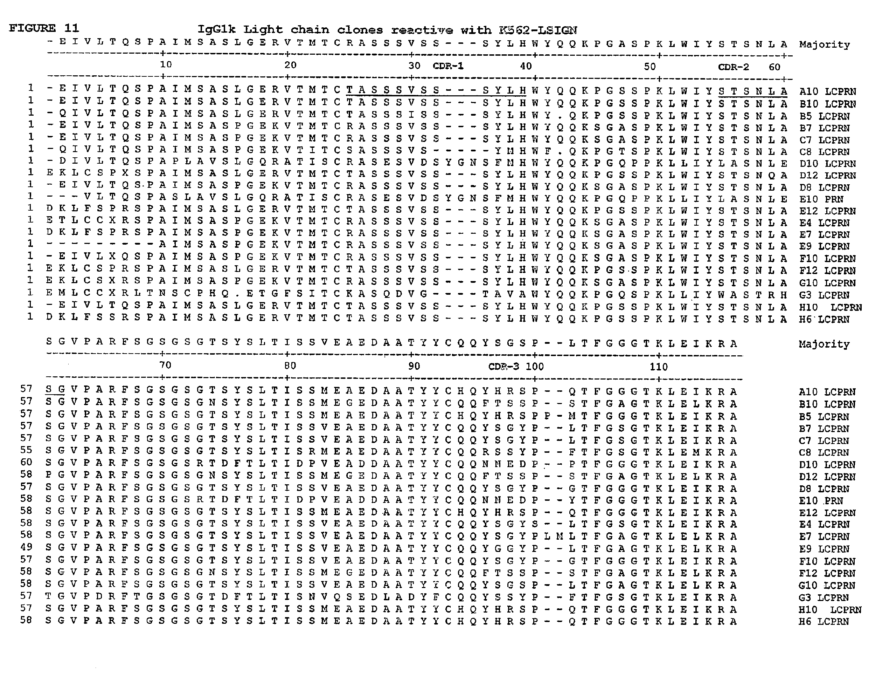

As set forth in Figure 11, additional clones that bind human DC-SIGNR were identified (SEQ. ID NOS: 96-115). These clones were found to have IgG1k light chain CDR3 regions with one of the following amino acid sequences: Q Y H R S P Q T (SEQ. ID NO: 116); C Q Q F T S S P S (SEQ. ID NO: 117); Q Q Y S GYP LT(SEQ. ID NO: 118); QQYS GYP GT(SEQ. ID NO: 119); HQYH R S P P M T (SEQ. ID NO: 120); Q Q R S S Y P F T (SEQ. ID NO: 121); Q Q Y S S Y P F T (SEQ. ID NO: 122); QQNNEDPPT (SEQ. ID NO: 123); Q Q Y S G Y S L T (SEQ. ID NO: 124); QQYSGYPLMLT (SEQ. ID NO: 125); Q Q Y G G Y P L T (SEQ. ID NO: 126); Q Q N N E D P Y T (SEQ. ID NO: 127); Q Q Y S G S P L T (SEQ. ID NO: 128). The CDR2 regions of these clones that bound to human DC-SIGNR were found to have one of the following amino acid sequences: S T S N L A S G (SEQ. ID NO: 129); L A S N L E S G (SEQ. ID NO: 130); S T S N Q A P G (SEQ. ID NO: 131); W A S T R H T G (SEQ. ID NO: 132).

Table 5 shows additional IgG2ak antibody clones selected based on their reactivity with cells expressing L-SIGN

**TABLE 5**

| **Ab clusters** | **Light chain** | **Heavy chain** | **Unique clones** |
|---|---|---|---|
| 1 | A12*, B11, | A12, C10, C12, H7 | A12, C12, H7 |
| | C12*,C6, E12, E8*, F10*, H7* | | |
| 2 | F6*, F12* | F12, F6 | F12, F6 |
| 3 | C10, G10, G5 | A3, F10, G5 | A3 |
| 4 | A4, B9 | A4, C7, D12 | A4 |
| 5 | A3 | A5, D8 | C7 |
| 6 | A5 | C5* | D12 |
| 7 | C7 | C6 | F10 |
| 8 | D8 | B9 | G5 |
| 9 | D12 | E12 | C5* |
| 10 | H6 | H6 | H6 |
| 11 | - | G10 | B9 |
| 12 | - | B11 | E12 |
| 13 | - | E8 | G10 |
| 14 | | | B11 |
| 15 | | | E8 |
| 16 | | | C6 |
| 17 | | | A5 |
| 18 | | | D8 |
| 19 | | | C10 |

| | | | |
|---|---|---|---|
| * These sequences contain a stop codon | | | |

Table 6 shows the reactivity of additional IgG2ak antibody clones with cells expressing LSIGN (Geometric Mean fluorescence) and recombinant L-SIGN and DC-SIGN proteins (OD values)

**TABLE 6**

| **IgG2ak** | **Geo. Mean Flourecence** | **Geo. Mean Flourecence** | **OD405** | **OD405** |
|---|---|---|---|---|
| Clone Name | K562 | K562/L-SIGN | L-SIGN | DC-SIGN |
| A3 | 2.8 | 15.4 | 3.5 | 1.0 |
| A4 | 2.8 | 12.1 | 3.5 | 0.1 |
| A5 | 1.8 | 59.8 | 3.5 | 0.1 |
| A12 | 2.3 | 23.4 | 3.5 | 0.8 |
| B9 | 3.5 | 31.4 | 3.5 | 0.1 |
| B11 | 3.4 | 14.2 | 3.5 | 0.6 |
| C5 | 3.3 | 13.9 | 3.5 | 0.6 |
| C6 | 2.6 | 13.2 | 3.5 | 0.6 |
| C7 | 2.5 | 21.2 | 3.5 | 0.1 |
| C10 | 2.8 | 25.8 | 3.5 | 0.7 |
| C12 | 2.9 | 23.3 | 3.5 | 1.0 |
| D8 | 3.2 | 17.3 | 3.5 | 0.1 |
| D12 | 2.3 | 41.0 | 3.5 | 0.1 |
| E8 | 2.9 | 11.2 | 3.5 | 0.4 |
| E12 | 3.5 | 19.4 | 3.5 | 0.7 |
| F6 | 2.7 | 13.8 | 3.5 | 0.6 |
| F10 | 2.6 | 18.3 | 3.5 | 1.0 |
| F12 | 2.0 | 9.5 | 3.5 | 0.5 |
| G5 | 3.1 | 10.7 | 3.5 | 0.4 |
| G10 | 2.6 | 21.5 | 3.5 | 0.7 |
| H6 | 3.2 | 12.7 | 3.5 | 0.6 |
| H7 | 2.1 | 11.5 | 3.5 | 1.0 |

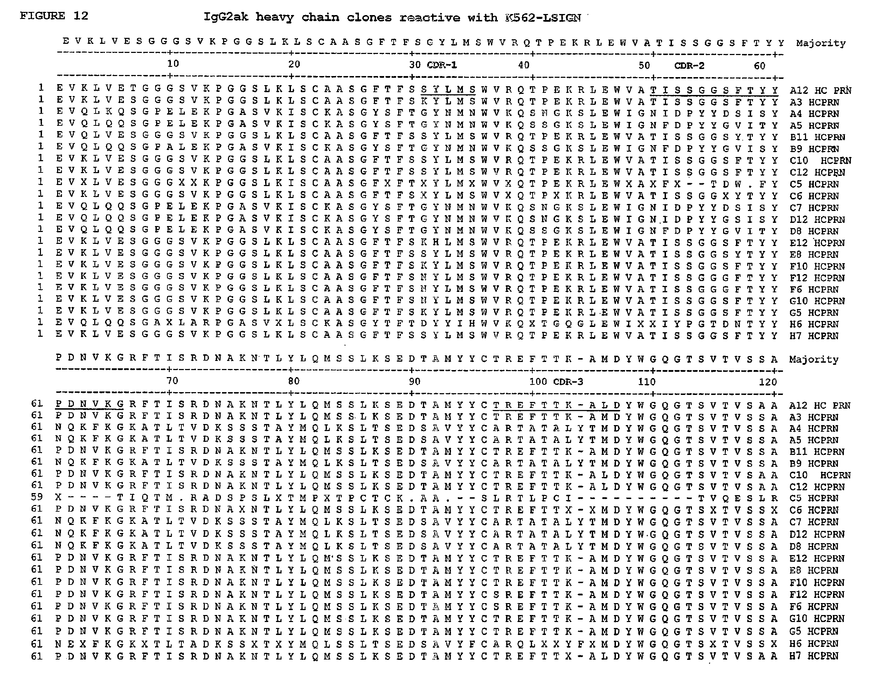

As set forth in Figure 12, additional heavy chain clones that bind human DC-SIGNR were identified (SEQ. ID NOS: 133-154). These clones were found to have IgG2ak heavy chain CDR3 regions with one of the following amino acid sequences: T R E F T T K A L D (SEQ. ID NO: 155); T R E F T T K A M D (SEQ. ID NO: 156); A R T A T A L Y T M D (SEQ. ID NO: 157); L R T L P C I (SEQ. ID NO: 158); S R E F T T K A M D (SEQ. ID NO: 159); A R Q L X X Y F X M D (SEQ. ID NO: 160). The CDR2 regions of these clones that bound to human DC-SIGNR were found to have one of the following amino acid sequences: T I S S G G S F T Y Y P D N V K G (SEQ. ID NO: 161); N I D P Y Y D S I S YN Q K F K G (SEQ. ID NO: 162); NFDPYYGVITYNQKFKG (SEQ. ID NO: 163); T I S S G G S Y T Y Y P D N V K G (SEQ. ID NO: 164); X F X T D W. F Y X T (SEQ. ID NO: 165); N F D P Y Y G V I S Y N Q K F K G (SEQ. ID NO: 166); T I S S G G G F T Y Y P D N V K G (SEQ. ID NO: 167); X I Y P G T D N T Y Y N E X F K G (SEQ. ID NO: 168).

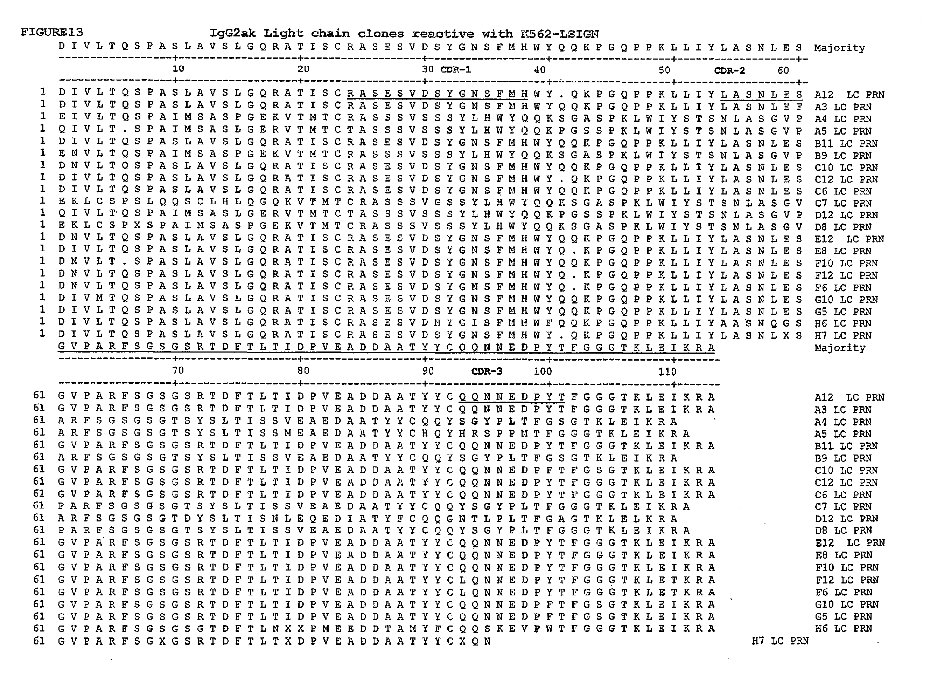

As set forth in Figure 13, additional light chain clones that bind human DC-SIGNR were identified (SEQ. ID NOS: 169-189). These clones were found to have IgG2ak light chain CDR3 regions with one of the following amino acid sequences: Q Q N N E D P Y T (SEQ. ID NO: 190); S G Y P L T F G S (SEQ. ID NO:191); H R S P P M T F G (SEQ. ID NO: 192); Q Q N N E D P F T (SEQ. ID NO: 193); Y S G Y P L T F G (SEQ. ID NO: 194); N T L P L T F G (SEQ. ID NO: 195); Q Q S K E V P W T (SEQ. ID NO: 196); L Q N N E D P Y T F (SEQ. ID NO: 197). The CDR2 regions of these clones that bound to human DC-SIGNR were found to have one of the following amino acid sequences: L A S N L E S (SEQ. ID NO: 198); L A S N L E F (SEQ. ID NO: 199); N L A S G V P (SEQ. ID NO: 200); N L A S G V (SEQ. ID NO: 201); A A S N Q G S (SEQ. ID NO: 202).
- Aspord C, Thivolet C (2002) Nasal administration of CTB-insulin induces active toleranceagainst autoimmune diabetes in non-obese diabetic (NOD) mice. Clin Exp Immunol 130:
- Atkinson MA, Kaufman DL, Campbell L, Gibbs KA, Shah SC, Bu DF, Erlander MG, Tobin AJ, Maclaren NK (1 992) Response of peripheral-blood mononuclear cells to glutamate decarboxylase in insulin-dependent diabetes. Lancet 339: 458-459.
- Atkinson MA, Leiter EH (1999) The NOD mouse model of type I diabetes: As good as it gets? Nat Med 6: 601-604.
- Atkinson MA, MacLaren NK, Luchetta R (1990) Insulitis and diabetes in NOD mice reduced by prophylactic insulin therapy. Diabetes 39: 956-960.
- Bach JF (2001) Immunotherapy of insulin-dependent diabetes mellitus. Curr Opin Immunol 13: 601-605.
- Baekkeskov S, Aanstoot HJ, Christgau S, Reetz A, Lolimena M, Cascalho F, Folli F, Richter-Oelsen H, Decamilli P (1990) Identification of the 64 K autoantigen in insulin-dependent diabetes as the GABA-synthesizing enzyme glutamic acid decarboxylase. Nature 347: 151 -156.
- Barrat FJ, Cua DJ, Boonstra A, Richards DF, Crain C, Savelkouf HF, de Waal-Malefyt R, Coffman RL, Hawrylowicz CM, O'Garra A (2002) In vitro generation of interleukin 10-producing regulatory CD4(+) T cells is induced by immunosuppressive drugs and inhibited by T helper type i (Thl) and Th2-inducing cytokines. J Exp Med 195: 603-616.
- Bashirova AA, Geijtenbeek TBH, van Duijnhoven GCF et al (2001) A dendritic cell-specific intercellular adhesion molecule 3-grabbing non-integrin (DC-SIGN) related protein is highly expressed on human liver sinusoidal endothelial cells and promotes HIV-1 infection. J Exp Med 193: 671-678.
- Bellmann K, Kolb H, Rastegar S Jee P, Scott FW (1998) Potential risk of oral insulin with adjuvant for the prevention of type I diabetes: a protocol effective in NOD mice may exacerbate disease in BB rats. Diabetologia 41 : 844-847.
- Bercovici N, Heurtier A, Vizier C, Pardigon N, Cambouris C, Desreumaux P, Liblau R (2000) Systemic administration of agonist peptide blocks the progression of spontaneous CD8-mediated autoimmune diabetes intrasgenic mice without bystander damage. J Immunol 165:202-210.
- Blana E, Carbone FR, Allison H, Miller JF, Heath WR (1996) Induction of autoimmune diabetes by oral administration of autoantigen. Science 274.: 1707-1709.
- Boitard C, Yasunami R, Dardenne M, Bach JF (1989) T cell mediated inhibition of the transfer of autoimmune diabetes in NOD mice. J Exp Med 169: 1669-1680.
- Calne R (1969) Induction of immunological tolerance by porcine liver allografts. Nature 223:427-476.
- Cameron MJ, Arreaza GA, Zucker P, Chensue SW, Strieter RM, Chakrabarti S, Delovitch TL(1997) IL-4 prevents insulitis and insulin-dependent diabetes mellitus in nonobese diabeticmice by potentiation of regulatory T helper-2 cell function. J Immunol 159: 4686-4692.
- Chaillous L, Lefevre H, Thivolet C, Boitard C, Lahlou N, Atlan Gepner C, Bouhanick B, Mognet A, Nicolino M, Carel J, Lecomte P, Marechaud R, Bougneres P, Charbonnel B, Sai P(2000) Oral insulin administration and residual beta-cell function in recent-onset type 1 diabetes: a multicentre randomized controlled trial. Lancet 356: 545-549.
- Chatenoud L, Primo J, Bach JF (1997) CD3 antibody-induced self tolerance in overtly diabetic NOD mice. J Immunol 158:2947-2954.
- Chatenoud L, Thervet E, Promo J, Bach JF (1994) Anti-CD3 antibody induces long-term remission of overt autoimmunity in nonobese diabetic mice. Proc Natl Acad Sci USA 89:3434-3438.
- Daniel D, Gill RG, Schloot N, Wegmann D (1995) Epitope specificity, cytokine production profile and diabetogenic activity of insulin-specific T cell clones isolated from NOD mice. Eur. J Immunol 25: 1056-1062.
- Dhodapkar MV, Steinman RM (2001) Antigen-specific inhibition of effector T cell function in humans after injection of immature dendritic cells. J Exp Med 193:233-238.
- Dhodapkar MV, Steinman RM (2002) Antigen-bearing immature dendritic cells induce peptide-specific CD8(+) regulatory T cells in vivo in humans. Blood 100:174-177.
- Diabetes Prevention Trial-Type I (DPT) Study Group (2002): Effects of parental insulin in relatives at high risk of type 1 diabetes mellitus. N Eng J Med 346: 1685-1691.
- Elias D, Cohen IR (1994) Peptide therapy for diabetes in NOD mice. Lancet 343: 704-706.
- Elias D, Meilin A, Ablamuntis V, Birk OS, Carmi P, Konen-Waisman S, Cohen IR (1997) Hsp6O peptide therapy of NOD mouse diabetes induces a Th2 cytokine burst and down regulates autoimmunity to various beta cell antigens. Diabetes 46: 758-764.
- Elias D, Reshef T, Birk OS, Van der Zee R, Walker MD, Cohen IR (1991) Vaccination against autoimmune mouse diabetes with a T cell epitope of the human 65 kDa heat shock protein. Proc Natl Acad Sci USA 88: 3088-3091.
- Engering A, Geijtenbeek TBH, van Vliet SJ et al (2002) The dendritic cell-specific adhesion receptor DC-SIGN internalizes antigen for presentation to T cells. J Immunol 168: 21 18-2126.
- Feutren G, Papoz L, Assan R et al (1988) Cyclosporin increases the rate and length of remissions in insulin-dependent diabetes of recent onset. Results of a multicenter double- blind trail. Lancet ii:I19-124.
- Geijtenbeek TBH, Groot PC, Nolte MA, van Vliet SJ, Gangaram-Panday ST, van Duijnhoven GCF, Kraal G, van Oosterhout AJM, van Kooyk Y (2002) Marginal zone macrophages express a murine homologue of DC-SIGN that captures blood-borne antigens in vivo. Blood 100: 2908-2916.
- ²Geijtenbeek TBH, Krooshoop DJEB, Bleijs DA et al (2000) DC-SIGN-ICAM-2 interaction mediates dendritic cell trafficking. Nat Immunol 1 : 252-257.
- ³Geijtenbeek TBH, Torensma R, van Vliet SJ, van Duijnhoven GCF, Adema GJ, van Kooyk Y, Figdor CG (2000) Identification of DC-SIGN, a novel dendritic cell-specific CAMPS receptor that supports primary immune responses. Cell 100: 575-585.
- Hawiger D, Inaba K, Dorsett Y, Guo M, Mahnke K, Rivera M, Ravetch JV, Steinman RM, Nussenzweig MC (2001) Dendritic cells induce peripheral T cell unresponsiveness under steady state conditions in vivo. J Exp Med 194:769-779.
- Heath WR, Carbone FR (2001) Cross-presentation, dendritic cells, tolerance and immunity. Annu Rev Immunol 19: 47-64.
- Herold KC, Hagopian W, Auger JA, Poumian-Ruiz E, Taylor L, Donaldson D, Gitelman SE, Harlan D, Xu D, Zivin RA, Bluestone JA (2002) Anti-CD3 monoclonal antibody in new-onset type I diabetes mellitus. N Eng J Med 346: 1692-1698.
- Homann D, Holz A, Bot A, Coon B, Wolfe T, Petersen J, Dyrberg TP, Grusby MJ, von Herrath MG (1 999) Autoreactive CD4+ T cells protect from autoimmune diabetes via bystander suppression using the IL-4/Stat6 pathway. Immunity 11: 463-472.
- Honeyman MC, Cram DS, Harrison LC (1993) Glutamic acid decarboxyase 67-reactive T cells: a marker of insulin-dependent diabetes. J. Exp. Med. 177:335-340.
- Huges S, Mougneau E Ferlin W, Jeske D, Hofman P, Homann D, Beaudoin L, Schrike C, von Herrath M, Lehuen A et al (2002) Tolerance to islet antigens and prevention from diabetes induced by limited apoptosis of pancreatic beta cells. Immunity 16:169-181.
- Hurlbert MS, Stallard JF, Furlanetto RW (2001) Symposium summary, Experimental Biology. J Investig Med 49:554-558.
- Hutchings PR, Cooke A (1 990) The transfer of autoimmune diabetes in NOD mice can be inhibited or accelerated by distinct cell populations present in normal splenocytes taken from young males. J Autoimmun 3: 175-1 85.
- Jonuleit H, Schmitt E, Schuler G, Knop J, Enk AH (2000) Induction of interleukin 10-producing, nonproliferating CD4 T cells with regulatory properties by repetitive stimulation with allogeneic immature human dendritic cells. J Exp Med 192: 121 3-1222.
- Kamei T, Callery MP, Flye MW (1990) Pretransplant portal venous administration of donor antigen and portal venous allograft drainage synergistically prolong rat cardiac allograft survival. Surgery 108: 41 5-421.
- Kaufman DL, Claire-Salzier M, Tian J, Forsthuber T, Ting GSP, Robinson P, Atkinson MA, Sercarz EE, Tobin AJ, Lehmann PV (1993) Spontaneous loss of T-cell tolerance to glutamic acid decarboxylase in murine insulin-dependent diabetes. Nature 366:69-72.
- Kaufman DL, Erlander MG, Clare-Salzer M., Atkinson MA, Maclaren NK, Tobin AJ (1992) Autoimmunity to two forms of glutamate decarboxylase in insulin-dependent diabetes mellitus. J. Clin. Invest. 89:283-292.
- King C, Mueller HR, Malo CM, Murali-Krishna K, Ahmed R, King E, Sarvetnick N (2002) Interleukin-4 acts at the locus of the antigen-presenting dendritic cell to counter-regulate cytotoxic CD8+ T cell responses. Nat Med 7:206-214.
- Klugewitz K, Blumenthal-Barby F, Schrage A, Knolle PA, Hamann A, Crispe IN (2002) Intramodulatory effects of the liver: deletion of activated CD4+ cells and suppression of IFN-γ producing cells after intravenous protein immunization. J Immunol 169: 2407-241 3.
- ¹Knolle P, Gerken G (2000) Local regulation of the immune response in the liver. Immunol Rev 174: 21-34.
- ²Knolle PA, Germann T, Treichel U, Uhrig A, Schmitt E, Hegenbarth S, Lohse AW, Gerken G (1999) Endotoxin down-regulates T cell activation by antigen-presenting liver sinusoidal endothelial cells. J Immunol 162: 1401 -1407.
- ³Knolle PA, Schmitt E, Jin S, Germann T, Duchmann R, Hegenbarth S, Gerken G, Lohse AW (1999) Induction of cytokine production in naive CD4 T cells by antigen-presenting murine liver sinusoidal endothelial cells but failure to induce differentiation toward Th1 cells. Gastroenterology 1 16:1428-1 440.
- Knook DL, Sleyster ECH (1976) Separation of Kupffer and endothelial cells of the rat liver by centrifugal elutriation. Exp Cell Res 99: 444-449.
- Kretz-Rommel A, Boelsterli UA (1995) Cytotoxic activity of T cells and non-T cells from diclofenac-immunized mice against cultured syngeneic hepatocytes exposed to diclofenca. Hepatology 22: 21 3-222.
- Kretz-Rommel A, Rubin RL (1997) A metabolite of the lupus-inducing drug procainamide prevents anergy induction in T cell clones. J Immunol 158: 4465-4470.
- Kurts C, Sutherland RM, Davey G, Li M, Lew AM, Blanas E, Carbone FR, Miller JFAP, Heath WR (1999) CD8 T cell ignorance or tolerance to islet antigens depends on antigen dose. Proc Natl Acad Sci USA 96: 12703-12707.
- Lafaille MACD and Lafaille JJ (2002) CD4+ regulatory T cells in autoimmunity and allergy. Curr Opin Immunol 14:771-778.
- Limmer A, Ohl J, Kurts C, Ljunggren HG, Reiss Y, Groettrup M, Momburg F, Arnold B, Knolle PA (2000) Efficient presentation of exogenous antigen by liver endothelial cells to CD8+ T cells results in antigen-specific T-cell tolerance. Nat Med 6: 1348-1354.
- Lohman T, Leslie RGD, Londei M (1996) T cell clones to epitopes of glutamic acid decarboxylase 65 raised from normal subjects and patients with insulin-dependent diabetes. J Autoimmun 9: 385-389.
- Lohse AW, Knolle PA, Bilo K, Uhrig A, Waldmann C, Ibe M, Schmitt E, Gerken G, Meyer Zum Buschenfelde KH (1 996) Antigen-presenting function and B7 expression of murine sinusoidal endothelial cells and Kupffer cells. Gastroenterology 110: 1 175-1 181.
- Ma SW, Zhao DL, Yin ZQ, Mukherjee R, Singh B, Qin HY, Stiller CR, Jevnikar AM (1997) Transgenic plants expressing autoantigens fed to mice to induce oral immune tolerance. Nat Med 3: 793-796.
- Mahnke K, Guo M, Lee S, Sepulveda H, Swain SL, Nussenzweig M, Steinman RM (2000) The dendritic cell receptor for endocytosis, DEC-205, can recycle and enhance antigen presentation via major histocompatiblity complex class II-positive lysosomal compartments. J Cell Biol 151 : 673-685.
- Miyazaki A et al (1985) Predominance of T lymphocytes in pancreatic islets and spleen of pre-diabetic non-obese (NOD) mice: a longitudinal study. Clin Exp Immunol 60:622-630.
- Notkins AL, Lernmark A (2001) Autoimmune type I diabetes: resolved and unresolved issues. J Clin Invest 108: 1247-1252.
- O'Reilly LA, Hutchings PR, Crocker PR, Simpson E, Lund T, Kiousses D, Takei F, Baird J, Cooke A (1991) Characterization of pancreatic islet cell infiltrates in NOD mice: effect of cell transfer and transgene expression. Eur J Immunol 21:1171-1180.
- Pakkala SV, Kurrer MO, Katz JD (1997) T helper 2 (Th2) cells induce acute pancreatitis and diabetes in immune-compromised nonobese diabetic (NOD) mice. J Exp Med 186:299-306.
- Park CG, Takahara K, Umemoto E et al (2001) Five mouse homologues of the human dendritic cell C-type lectin, DC-SIGN. Int Immunol 13: 1283-1290.
- Parving HH, Tarnow L, Nielsen F, Rossing P, Mandrup-Poulsen T, Osterby R, Nerup J (1999) Cyclosporine nephrotoxicity in type 1 diabetic patients. A 7-year follow-up study. Diabetes Care 22: 478-483.
- Pohlmann S, Soilleux EJ, Baribaud F et al(2001) DC-SIGNR, a DC-SIGN homologue expressed in endothelial cells, binds to human and simian immunodeficiency viruses and activates infection in trans. Proc Natl Acad Sci USA 98: 2670-2675.
- Poul MA, Becerril B, Nielsen UB, Morisson P, Marks JD (2000) Selection of tumor specific internalizing human antibodies from phage libraries. J Mol Biol 301: 1149-1161.
- Pozzili P, Pitocco D, Visalli N, Cavall MG, Buzzetti R, Crino A, Spera S, Suraci C, Multari G, Cervoni M (2000) No effect of oral insulin on residual beta-cell function in recent-onset type I diabetes (the IMDIAB VII). Diabetologia 43: 1000-1004.
- Pugliese A, Brown D, Garza D, Murchison D, Zeller M, Redondo M, Diez J, Eisenbarth GS, Patel DD, Ricordi C (2001) Self-antigen presenting cells expressing diabetes-associated autoantigens exist in both thymus and peripheral lymphoid organs. J Clin Invest 107: 555- 564.
- Quinn A, McInerney MF, Sercarz EE (2001) MHC Class I-restricted determinants on the glutamic acid decarboxylase 65 molecule induce spontaneous CTL activity. J Immunol 167: 1748 1757.
- Raz I, Elias D, Avron A, Tamir M, Metzger M, Cohen IR (2001) P-cell function in new-onset type 1 diabetes and immunomodulation with a heat-shock protein peptide (DiaPep 277): a randomized, double-blind phase II trail. Lancet 358: 1749-1 753.
- Reijonen H, Novak EJ, Kochik S, Henninger A, Liu AW, Kwok WW, Nepom GT (2002) Detection of GAD65-specific T cells by major histocompatibility complex class II tetramers in type I diabetic patients and at-risk subjects. Diabetes 51: 1375-1382.
- Roep BO, Arden SD, de Vries RRP, Hutton JC (1990) T cell clones from a type-I diabetes patient respond to insulin secretory granule proteins. Nature 345:632-634.
- Rohane PW, Shimada A, Kim DT, Edwards CT, Charlton B, Shultz LD, Fathman CG (1995) Islet-infiltrating lymphocytes from prediabetic NOD mice rapidly transfer diabetes to NOD-scid/scid mice. Diabetes 44: 550-554.
- Roncarolo MG, Levings MK, Traversi C (2001) Differentiation of T regulatory cells by immature dendritic cells. J Exp Med 193:F5-F9.
- Rubinstein D, Roska AK, Lipsky PE (1986) Liver sinusoidal lining cells express class II major histocompatibility antigens but are poor stimulators if fresh allogenic lymphocytes. J Immunol 137: 1803-3818.
- Sempe P, Richard MF, Bach JF, Boitard C (1994) Evidence of CD4+ regulatory T cells in the non-obese diabetic male mouse. Diabetologica 37:337-343.
- Skyler J, Rabinovitch A (1992) Effects of cyclosporine in recent onset type I diabetes mellitus. Effects on islet beta cell function. Miami Cyclosporine Diabetes Study Group. J Diabetes 6: 77- 88.
- Smith JA, Tang Q, Bluestone JA (1 998) Partial TCR signals delivered by Fc-R-nonbinding anti-CD3 monoclonal antibodies differentially regulate individual Th subsets. J Immunol 160:4841-4849.
- Smith JA, Tso JY, Clark MR, Cole MS, Bluestone JA (1997) Nonmitogenic anti-CD3 antibodies deliver a partial T cell receptor signal and induce clonal anergy. J Exp Med 185: 1413-1422.
- Soilleux EJ, Batten R, Trowsdale J (2000) Cutting Edge: DC-SIGN; a related gene, DC-SIGNR; and CD23 form a cluster on 19~13. J Immunol 165: 2937-2942.
- Thornton AM, Shevach EM (1998) CD4+CD25+ immunoregulatory T cells suppress polyclonal T cell activation in vitro by inhibiting interleukin 2 production. J Exp Med 188: 287-296.
- Thorton AM, Shevach EM (2000) Suppressor function of CD4+CD25+ immunoregulatory T cells is antigen nonspecific. J Immunol 164: 183-1 90.
- Tian JD, Clare-Salzler M, Herschenfeld A, Middleton B, Newman D, Mueller R, Arita S, Evans C, Atkinson MA, Mullen Y, Sarvetnick N, Tobin AJ, Lehmann PV, Kaufman DL (1996) Modulating autoimmune responses to GAD inhibits disease progression and prolongs islet graft survival in diabetes-prone mice. Nat Med 2: 1348-1 353.
- Tian J, Atkinson MA, Clare-Salzer M, Herschenfeld A, Forsthuber T, Lehmann PV, Kaufman DL (1996) Nasal administration of glutamate decarboxylase (GAD65) peptides induces Th2 responses and prevents murine insulin-dependent diabetes. J Exp Med 183: 1561 -1 567.
- Tisch R., Yang XD, Singer SM, Liblau R, Fugger L, McDevitt H (1993) Immune response to glutamic acid decarboxylase correlates with insulitis in non-obese diabetic mice. Nature 366: 72-75
- Tisch R, Wang B, Atkinson MA, Serreze DV, Friedline R (2001) A glutamic acid decarboxylase 65-specific Th2 cell clone immunoregulates autoimmune diabetes in nonobese diabetic mice. J Immunol 166: 6925-2936.
- Tisch R, Wang B, Serreze DV (1 999) Induction of glutamic acid decarboxylase 65-specific Th2 cells and suppression of autoimmune diabetes at late stages of disease is epitope dependent. J Immunol 163: 1178-1187.
- Wakkach A, Cottrez F, Groux H (2001) Differentiation of regulatory T cells is induced by CD2 co-stimulation. J Immunol 167:3107-3113.
- Weiner HL (2001) Oral tolerance: immune mechanisms and the generation of Th3-type TGF-beta secreting regulatory T cells. Microbes Infect 3: 947-954.
- Wen L, Wong FS, Burkly L, Altieri M, Mamalaki C, Kioussis D, Flavell RA, Sherwin RS (1998) Induction of insulitis by glutamic acid decarboxylase peptide-specific and CD4(+) T cells from human DQ transgenic mice. J Clin Invest 102: 947-957.
- Wong FS, Visintin I, Wen L, Flavell RA, Janeway CA Jr (1996) CD8 T cell clones from young nonobese diabetic (NOD) islets can transfer rapid onset diabetes in NOD mice in the absence of CD4 cells. J Exp Med 183: 67-76.
- Yagi H, Matsumoto M, Kunimoto K, Kawaguchi J, Makino S, Harada M (1992) Analysis of parts of CD4+ and CD8+ T cells in autoimmune diabetes of NOD mice using transfer to NOD athymic mice. Eur J Immunol 22: 2387-2393.
- Yamigawa S, Gray JD, Hashimoto S, Horwitz DA (2001) A role for TGF-beta in the generation and expansion of CD4+CD25+ regulatory T cells from human peripheral blood. J Immunol 166: 7282-7289.
- Yang R, Liu Q, Grosfeld JL, Pescovitz MD (1994) Intestinal venous drainage through the liver is a prerequisite for oral tolerance induction. J Pediatr Surg 29: 1145-1148.
- Zechel MA, Elliott JF, Atkinson MA, Singh B (1998) Characterization of novel T-cell epitopes on 65 kDa and 67 kDa glutamic acid decarboxylase relevant in autoimmune responses in NOD mice. J Autoimmun 1 :83-95.
- Zekzer D, Wong SF, Ayalon 0, Millet I, Altieri M, Shigeki S, Solimena M, Sherwin R (1998) GAD-reactive CD4+Thl cells induce diabetes in NOD/SCID mice. J Clin. Invest 101 :68-73.
- Zhang ZJ, Davidson L, Eisenbarth G, Weiner HL (1991) Suppression of diabetes in nonobese diabetic mice by oral administration of porcine insulin. Proc Natl Acad Sci USA 88:10252- 10256.

## Claims

1. An antibody/peptide construct comprising: (i) an antibody that recognizes L-SIGN and (ii) a peptide linked to the antibody, wherein the peptide is an autoantigen, wherein the autoantigen is selected from the group consisting of glutamic acid decarboxylase (GAD), an epitope of GAD, insulin, an epitope of insulin, heat shock protein (HSP), an epitope of HSP and β cell antigens.

2. The antibody/peptide construct according to claim 1, wherein i) the L-SIGN is a human L-SIGN and ii) the peptide is a human peptide.

3. A composition comprising an antibody/peptide construct in accordance with any one of claim 1 or 2 and a pharmaceutically acceptable carrier.

4. A method for recombinantly producing engineered antibodies, the method comprising linking an antibody that recognizes L-SIGN to an autoantigen, wherein the autoantigen is selected from the group consisting of glutamic acid decarboxylase (GAD), an epitope of GAD, insulin, an epitope of insulin, heat shock protein (HSP), an epitope of HSP and β cell antigens.

5. The method according to claim 4, wherein i) the L-SIGN is a human L-SIGN and ii) the peptide is a human peptide.

## Patentansprüche

1. Antikörper/Peptid-Konstrukt umfassend: (i) einen Antikörper, der L-SIGN erkennt, und (ii) ein Peptid, das mit dem Antikörper verbunden ist, wobei das Peptid ein Autoantigen ist, wobei das Autoantigen Glutaminsäure-Decarboxylase (GAD), ein Epitop von GAD, Insulin, ein Epitop von Insulin, Hitzeschockprotein (HSP), ein Epitop von HSP oder ein β-Zellen-Antigen ist.

2. Antikörper/Peptid-Konstrukt nach Anspruch 1, wobei i) das L-SIGN ein menschliches L-SIGN ist und ii) das Peptid ein menschliches Peptid ist.

3. Zusammensetzung umfassend ein Antikörper/Peptid-Konstrukt nach einem der Ansprüche 1 oder 2 und einen pharmazeutisch verträglichen Träger.

4. Verfahren zur rekombinanten Herstellung technisch veränderter Antikörper, wobei das Verfahren das Verbinden eines Antikörpers, der L-SIGN erkennt, mit einem Autoantigen umfasst, wobei das Autoantigen Glutaminsäure-Decarboxylase (GAD), ein Epitop von GAD, Insulin, ein Epitop von Insulin, Hitzeschockprotein (HSP), ein Epitop von HSP oder ein β-Zellen-Antigen ist.

5. Verfahren nach Anspruch 4, wobei i) das L-SIGN ein menschliches L-SIGN ist und ii) das Peptid ein menschliches Peptid ist.

## Revendications

1. Construction anticorps/peptide comprenant : (i) un anticorps reconnaissant L-SIGN et (ii) un peptide lié à l'anticorps, dans laquelle le peptide est un autoantigène, dans laquelle l'autoantigène est sélectionné dans le groupe constitué d'une acide glutamique décarboxylase (GAD), un épitope de GAD, l'insuline, un épitope de l'insuline, une protéine de choc thermique (HSP), un épitope de HSP et des antigènes de cellules β.

2. Construction anticorps/peptide selon la revendication 1, dans laquelle i) le L-SIGN est un L-SIGN humain et ii) le peptide est un peptide humain.

3. Composition comprenant une construction anticorps/peptide selon l'une quelconque des revendications 1 ou 2 et un véhicule acceptable sur le plan pharmaceutique.

4. Méthode de production d'anticorps modifiés par voie recombinante, la méthode comprenant la liaison d'un anticorps reconnaissant L-SIGN à un autoantigène, dans laquelle l'autoantigène est sélectionné dans le groupe constitué d'une acide glutamique décarboxylase (GAD), un épitope de GAD, l'insuline, un épitope de l'insuline, une protéine de choc thermique (HSP), un épitope de HSP et des antigènes de cellules β.

5. Méthode selon la revendication 4, dans laquelle i) le L-SIGN est un L-SIGN humain et ii) le peptide est un peptide humain.
